# EUROPEAN PATENT SPECIFICATION

(11) **EP 3 675 875 B1**
(45) Date of publication and mention of the grant of the patent: **11.12.2024**
(21) Application number: 18852224.7
(22) Date of filing: 28.08.2018
(51) Int. Cl.: A61K 47/69, A61K 33/26, A61K 9/14, A61K 31/155, B82Y 5/00, A61K 31/7105, A61K 38/00, A61K 9/00, A61K 9/06, A61K 9/107, A61K 9/12, A61K 33/244, A61P 31/00, B82Y 30/00

(54) **CORE-SHELL PARTICLES COMPRISING METAL OXIDE AND LANTHANIDE ELEMENT**
KERN-SCHALE-TEILCHEN MIT METALLOXID UND LANTHANIDELEMENT
PARTICULES NOYAU-ENVELOPPE COMPRENANT UN OXYDE MÉTALLIQUE ET UN ÉLÉMENT LANTHANIDE

(30) Priority: 28.08.2017 US 201762550726 P
(43) Date of publication of application: 08.07.2020
(73) Proprietor: Bar-Ilan University, 5290002 Ramat Gan (IL)
(72) Inventor: LELLOUCHE, Jean Paul, 7748028 Ashdod (IL); MICHAELI, Shulamit, 5552102 Kiryat Ono (IL); ISRAEL, Limor liron, 7174705 Modi'in-Maccabim-Re'ut (IL); HAREL, Yifat, 5820329 Holon (IL); DOLITZKY, Avishay, 4937965 Petah Tikva (IL); OSTROVSKY, Stella, 4252025 Netanya (IL)
(74) Representative: Boult Wade Tennant LLP
(86) International application number: PCT/IL2018/050951
(87) International publication number: WO 2019/043701

(56) References cited:
- WO-A1-2014/147608
- ISRAEL LIRON L. ET AL: "Ce 3/4+ cation-functionalized maghemite nanoparticles towards siRNA-mediated gene silencing", JOURNAL OF MATERIALS CHEMISTRY. B, vol. 2, no. 37, 1 January 2014 (2014-01-01), GB, pages 6215 - 6225, XP055785401, ISSN: 2050-750X, Retrieved from the Internet <URL:https://pubs.rsc.org/en/content/articlepdf/2014/tb/c4tb00634h> DOI: 10.1039/C4TB00634H
- ISRAEL LIRON L ET AL: "Supporting Information Ce 3/4+ Cation-Functionalized Maghemite Nanoparticles Towards siRNA- Mediated Gene Silencing", JOURNAL OF MATERIALS CHEMISTRY B, 1 January 2014 (2014-01-01), pages SI - 1, XP055785402, Retrieved from the Internet <URL:http://www.rsc.org/suppdata/tb/c4/c4tb00634h/c4tb00634h1.pdf> [retrieved on 20210315]
- UNCITI-BROCETA JUAN D. ET AL: "Specific Cell Targeting Therapy Bypasses Drug Resistance Mechanisms in African Trypanosomiasis", PLOS PATHOGENS, vol. 11, no. 6, 25 June 2015 (2015-06-25), US, pages e1004942, XP055911107, ISSN: 1553-7366, DOI: 10.1371/journal.ppat.1004942
- FUSAI T. ET AL: "Action of pentamidine-bound nanoparticles against Leishmania on an in vivo model", PARASITE, vol. 1, no. 4, 1 December 1994 (1994-12-01), FR, pages 319 - 324, XP055911108, ISSN: 1252-607X, Retrieved from the Internet <URL:https://www.parasite-journal.org/articles/parasite/pdf/1994/05/parasite1994014p319.pdf> DOI: 10.1051/parasite/1994014319
- DL: ISRAEL , L. L. ET AL.: "Acute in vivo toxicity mitigation of PEI-coated maghemite nanoparticles using controlled oxidation and surface modifications toward siRNA delivery", ACS APPLIED MATERIALS & INTERFACES, vol. 7, no. 28, 29 June 2015 (2015-06-29), pages 15240 - 15255, XP055581469, Retrieved from the Internet <URL:https://pubs.acs.org/doi/abs/10.1021/acsami.5b02743>
- ISRAEL , L. L. ET AL.: "Ultrasound-Mediated Surface Engineering of Theranostic Magnetic Nanoparticles: An Effective One-Pot Functionalization Process Using Mixed Polymers for siRNA Delivery", J NANOMED NANOTECHNOL, vol. 7, no. 385, 23 January 2016 (2016-01-23), pages 2, XP055581473, Retrieved from the Internet <URL:https://pdfs.semanticscholar.org/09ab/8816f2dffe2d9fe651e04a4d8868e701afbb.pdf>
- LELLOUCHE, E. ET AL.: "Maghemite-containing PLGA-PEG-based polymeric nanoparticles for siRNA delivery: toxicity and silencing evaluation", RSC ADVANCES, vol. 7, no. 43, 19 May 2017 (2017-05-19), pages 26912 - 26920, XP055581475, Retrieved from the Internet <URL:https://pubs.rsc.org/en/content/articlehtml/2013/ra/c7ra00517b>

## Description

### FIELD OF INVENTION

This invention, in some embodiments thereof, relates to a composition comprising at least one particle, having a core comprising metal oxide, and a shell comprising an ion of a lanthanide element, wherein the lanthanide element is linked to a polymer and to pentamidine, and wherein a weight ratio of an active agent to the metal oxide is in the range of from 1:2 to 3:2, respectively.

### BACKGROUND OF THE INVENTION

Superparamagnetic iron oxide nanoparticles with appropriate surface chemistry can be used for drug delivery, and imaging techniques such as magnetic resonance imaging (MRI). Targeted drug delivery can be used to treat and to improve therapy efficacy of many diseases, such as cancer and leishmania.

Visceral leishmaniasis (VL) also known as Kala Azar causes death in developing countries and it is caused by either *Leishmania donovani* or *L. infantum* parasites. The current treatment is painful and often the wound is replaced with a scar that requires a plastic surgery. Pentamidine is an anti-trypanosomal drug used for the treatment of leishmania, *Cryptosporidium, Giardia lamlia, Toxoplasma gondii,* African trypanosomiasis, *Pneumocystic carinii* and *Pneumocystis pneumonia,* a common infection in HIV patients.

Nano-scale carriers are amenable to reduce the amount of drug injected and to circumvent resistance to the drug since the drug is delivered into the cell *via* endocytosis and not by binding to a specific ligand.

Design of Experiments (DoE) is a commonly applied technique for planning and analyzing experiments, allowing the use of a minimum number of experiments, allowing to systematically vary several experimental parameters simultaneously while capturing as much information as possible. In general usage, DoE is the design of any information-gathering exercises where variation is present, whether under the full control of the experimenter or not. In the design of experiments, the experimenter is often interested in the effect of some process or intervention on some objects. Design of experiments is thus a discipline that which has proved suitable to apply in bioprocessing.

Israel, Liron & Lellouche, Emmanuel & Kenett, Ron & Green, Omer & Michaeli, Shulamit & Lellouche, Jean-Paul. (2014). Ce3/4+ Cation-Functionalized Maghemite Nanoparticles Towards siRNA-Mediated Gene Silencing. J. Mater. Chem. B. 2. 10.1039/C4TB00634H, discloses Pre-formed Massart magnetite (Fe₃O₄) nanoparticles (NPs) have been successfully modified by positively charged lanthanide Ce(III/IV) cations/[CeL*ₙ*]^{3/4+} complexes by using a strong mono-electronic Ceric Ammonium Nitrate oxidant (CAN) as a Ce donor. Israel, Liron & Lellouche, Emmanuel & Ostrovsky, Stella & Yarmiayev, Valeria & Bechor, Moshe & Michaeli, Shulamit & Lellouche, Jean-Paul. (2015). Acute in Vivo Toxicity Mitigation of PEI-Coated Maghemite Nanoparticles Using Controlled Oxidation and Surface Modifications toward siRNA Delivery. ACS applied materials & interfaces. 7. 10.1021/acsami.5b02743 discloses the fabrication of core maghemite nanoparticles (NPs) that enabled the obtainment of colloid particles with a view to a high-level nanoparticle (NP) surface doping by Ce(III/IV). WO2014147608 discloses a nanoparticle, which has a metal oxide core and a cerium shell wherein the weight ratio of the cerium within the shell to the metal oxide in the core is at least 1%. Israel, Liron & Lellouche, Emmanuel & Greneche, Jean-Marc & Bechor, Moshe & Michaeli, Shulamit & Lellouche, Jean-Paul. (2016). Ultrasound-Mediated Surface Engineering of Theranostic Magnetic Nanoparticles: An Effective One-Pot Functionalization Process Using Mixed Polymers for siRNA Delivery. Journal of Nanomedicine & Nanotechnology. 7. 2157-7439. 10.4172/2157-7439.1000385 discloses that the versatility of the Ce cation-doped maghemite NPs fabrication process mediated by high-power ultrasound (US) enabled the development of a new one-step time-saving US-driven variant fabrication of corresponding polymer/organic species-grafted NPs. ellouche, Emmanuel & Locatelli, Erica & Israel, Liron & Naddaka, Maria & Kurlander, Ella & Michaeli, Shulamit & Lellouche, Jean-Paul & Comes-Franchini, Mauro. (2017). Maghemite-containing PLGA-PEG-based polymeric nanoparticles for siRNA delivery: Toxicity and silencing evaluation. RSC Adv.. 7. 26912-26920. 10.1039/C7RA00517B discloses incorporation of PEI into poly(D,L-lactide-co-glycolide)-poly(ethylene glycol) (PLGA-b-PEG) particles.

Juan D. Unciti-Broceta et al. 2015. (Juan D. Unciti-Broceta et al. 2015, Specific Cell Targeting Therapy Bypasses Drug Resistance Mechanisms in African Trypanosomiasis. Plos Pathogens. (https://doi.org/10.1371/journal.ppat.1004942)) provides nanoparticles of chitosan loaded with the trypanocidal drug pentamidine and coated by a single domain nanobody that specifically targets the surface of African trypanosomes. Once loaded into this nanocarrier, pentamidine enters trypanosomes through endocytosis instead of via classical cell surface transporters. The curative dose of pentamidine-loaded nanobody-chitosan nanoparticles was found to be 100-fold lower than pentamidine alone in a murine model of acute African trypanosomiasis.

T. Fusai et al. 1994. (T. Fusai et al. 1994. Action of pentamidine-bound nanoparticles against Leishmania on an in vivo model. Parasite 1994 Dec;1(4):319-24. doi: 10.1051/parasite/1994014319) provides that pentamidine bound nanoparticles (100 microM), enabled 77% amastigote reduction relative to the control group.

### SUMMARY OF THE INVENTION

According to some embodiments of the present invention, there is provided a composition comprising at least one particle, said at least one particle having a core comprising metal oxide, and a shell comprising an ion of a lanthanide element, wherein the lanthanide element is linked to a polymer and to the active agent pentamidine and wherein the weight ratio of the active agent to the metal oxide is in the range of from 1:2 to 3:2, respectively.

In some embodiments, the metal is iron (III) oxide in the form of gamma phase. In some embodiments, the metal oxide is magnetite (Fe₃O₄) or maghemite (gamma-Fe₂O₃; γ-Fe₂O₃).

In some embodiments, the lanthanide element is selected from the group consisting of: lanthanum, cerium, praseodymium, neodymium, promethium, samarium, europium, gadolinium, terbium, dysprosium, holmium, erbium, thulium, ytterbium, lutetium, or any combination thereof.

In some embodiments, the polymer is selected from the group consisting of: polyethylenimine (PEI), poly-L-lysine, chitosan, poly-L-arginine, dendrimeric polyamidoamine (PAMAM), polyacrylate, polyphosphate, polyethyleneglycol (PEG), and any derivative or combination thereof.

In some embodiments, the molecular weight of the polymer is in the range of from 500 Da to 60 KDa.

In some embodiments, there is provided a pharmaceutical product comprising the disclosed composition further comprising a pharmaceutically acceptable excipient. In some embodiments, the pharmaceutical product is formulated for systemic or topical administration. In some embodiments, the pharmaceutical product is formulated for parenteral, mucosal, nasal or oral administration. In some embodiments, the pharmaceutical product is for treating infectious diseases or disorders.

In some embodiments, the pharmaceutical product is disease or disorder is associated with parasitic infection. In some embodiments, the product is in the form of paste, cream, lotion, foam, gel, emulsion, an ointment, cream and soap.

According to an aspect of some embodiments of the present invention, there is provided a method for obtaining a composition comprising at least one particle, having a core comprising metal oxide, and a shell comprising an ion of a lanthanide element, wherein the ion of the lanthanide element has linked thereto a polymer and pentamidine, the method comprising the steps of:
(a) mixing a solvent, said lanthanide element, and a plurality of particles of said metal oxide, thereby obtaining a suspension;
(b) adding the polymer and the pentamidine to the suspension.

In some embodiments, the mixing step is performed under ultrasonic agitation.

In some embodiments, the polymer and the active are added simultaneously.

### BRIEF DESCRIPTION OF THE DRAWINGS

In the drawings:
**Figure 1** illustrates a scheme demonstrating the fabrication of polyethylenimine /pentamidine-ceric ammonium nitrate-γ-Fe₂O₃ (PEI/Penta-CAN-*γ*-Fe₂O₃) nanoparticles (NPs) ("/" denotes: and/or).
**Figure 2** illustrates the molecular structure of PEI (right) and pentamidine salt (left).
**Figure 3** presents a bar graph [each quartet in the bar graphs presents from left to right as follow: (1) promastigote cells treated with 0.375 µg/ml; (2) promastigote cells treated with 0.5 µg/ml; (3) amastigote cells treated with 0.5 µg/ml; and (4) MTT assay] demonstrating the viability of promastigotes, amastigotes and monocytes cells (MTT assay) following treatment with various NPs (as listed in Table 1 below) versus to control.
**Figure 4** presents a bar graph demonstrating the percentage of *Trypanosoma brucei* cells after 24 hours of incubation with *CAN-γ-Fe₂O₃* (right bar graph in each doublet) or after encapsulation of these NPs by 25 KDa PEI, providing *conPEI₂₅-CAN-γ-Fe₂O₃* NPs, (left bar graph in each doublet) compared to control ("con" denotes the contact mode of cerium coordinated to PEI polymeric phase). ***P value < 0.0001 by two-way ANOVA.
**Figure 5** presents a bar graph demonstrating the percentage of viable cells of various leishmanial strains [each triplet of the bar graphs presents from left to right as follow: (1) *L*. *donovani*; (2) *L. tropica;* and (3) *L. major*] after 24 hours of incubation with *conPEI₂₅-CAN-γ-Fe₂O₃* NPs, compared to control. ***P value < 0.0001.
**Figure 6** presents images demonstrating lysosomal bursting in *T. brucei* cells following treatment with *conPEI₂₅-CAN-γ-Fe₂O₃* NPs (right image) versus control (left image).
**Figure 7** presents a bar graph demonstrating the percentage of cells with lysosome before and after treatment of 0.75 µg/ml *conPEI₂₅-CAN-γ-Fe₂O₃* NPs. ***P value < 0.0001
**Figure 8** presents live imaging of lysosomal bursting in *T. brucei* procyclic cells following treatment with *conPEI₂₅-CAN-γ-Fe₂O₃* NPs (right image) versus control (left Image). Cells express fluorescent histone protein.
**Figure 9** presents images demonstrating lysis of *L. donovani* cells following treatment with *conPEI₂₅-CAN-γ-Fe₂O₃* NPs (right image) vis-à-vis the control (left image).
**Figure 10** presents a bar graph demonstrating human macrophage cell viability following treatment with various concentrations of *_{con}PEI₂₅-CAN-γ-Fe₂O₃* NPs compared to control (MTT assay).
**Figure 11** presents a schematic of the experimental setup for testing NP effects on amastigote infected macrophages.
**Figure 12** illustrates four images of amastigotes in macrophages infected with *L*. *donovani*, both control cells (left images; arrows pointing to fluorescent *L. donovani* cells) and cells after incubation with *conPEI₂₅-CAN-γ-Fe₂O₃* NPs (right images) are shown.
**Figure 13** presents a bar graph demonstrating the number of parasites containing macrophages per 30 cells after infection with *leishmania donovani* and after treatment of 0.75 µg/ml *conPEI₂₅-CAN-γ-Fe₂O₃* NPs as compared to control.
**Figure 14** presents parasite infected cell viability using hybrid pentamidine/PEI-grafted NPs (1132-penta) compared to the *_{con}*PEI₂₅-CAN-*γ*-Fe₂O₃ NPs (1108) and oxidized _{con}PEI₂₅-ox-γ-Fe₂O₃ NPs (1022-ox).
**Figure 15** presents a bar graph demonstrating the percentage of cells with DNA fragmentations (Sub-G1) after treatment with *_{con}*PEI₂₅/Penta-CAN-γ-Fe₂O₃ with various pentamidine/Fe ratios compared to the original effective *conPEI₂₅-CAN-γ-Fe₂O₃* NPs (1108).
**Figure 16A-16B** present bar graphs showing cell viability including macrophages (left bar; ^), promastigotes (middle bar; #) and amastigotes cells (right bar; @) following treatment with (**16A**) *_{con}*PEI₂₅/Penta-CAN-γ-Fe₂O₃ with various pentamidine/Fe ratios and (**16B**) optimized forms of the NPs.
**Figure 17** presents a bar graph showing the percentage of surviving promastigotes of L. donovani after incubation with varying concentrations (the 4 bars for each NP sample are, from left to right, a concentration of 0.1, 0.25, 0.375, and 0.5 µg/ml. The graph represents mean of three different biological repeats and the statistical significance is of student t-test and the p-values are <0.05 *; <0.01 **; <0.001 ***.
**Figures 18A-18D** present pareto charts of PDI (**18A**), pentamidine/Fe ratio (**18B**), amastigote (**18C**) and MTT assay (**18D**) for four conditions and their combination: solvent (A), pentamidine ratio to Fe (B), temperature (C), and reaction time (hours) (D).
**Figures 19A-19D** present main effects plots for amastigotes (**19A**), MTT assay (**19B**), dynamic light scattering (DLS) (**19C**) and zeta potential (**19D**) for four conditions: solvent, pentamidine ratio to Fe, temperature, and reaction time (hours).
**Figures 20A-20F** present contour plots for amastigote assay with varying temperature and reaction time (**20A**), DLS size with varying pentamidine ration to Fe and temperature (**20B**), zeta potential with varying pentamidine ratio to Fe and temperature (**20C**), PDI with varying pentamidine ratio to Fe and temperature (**20D**), pentamidine ratio to Fe with varying temperature and reaction time (**20E**) and amastigote assay with varying pentamidine ration to Fe and zeta potential (**20F**).
**Figure 21A-21B** present interaction plots for (**21A**) amastigote assay and (**21B**) MTT assay comparing solvent, pentamidine ratio, temperature, and reaction time.
**Figure 22** presents statistically designed experiment (DoE)-optimized prediction for fabrication conditions as further described hereinbelow.
**Figures 23A-23B** present thermogravimetric analyses (TGA) (**23A**) and first derivatives (**23B**) of DoE-optimized *_{con}*PEI₂₅/Penta-CAN-*γ*-Fe₂O₃ (line 1), *_{con}*PEI₂₅-CAN-*γ-*Fe₂O₃ (line 2), DoE-optimized *_{con}*PEI₂₅-CAN-γ-Fe₂O₃ NPs (line 3), and pentamidine (line 4).
**Figures 24A-24D** present Fourier transform infrared spectroscopy (FTIR) of: DoE-optimized *_{con}*PEI₂₅/Penta-CAN-*γ*-Fe₂O₃ NPs (**24A**), pentamidine (**24B**), branched-PEI₂₅ (**24C**) and CAN-maghemite NPs (**24D**).
**Figures 25A-25B** present transmission electron microscopy (TEM) image (**25A**) and a histogram showing size distribution by TEM (**25B**) of DoE-optimized *_{con}*PEI₂₅/Penta-CAN-γ-Fe₂O₃ NPs.
**Figure 26** presents bar graphs of blood toxicology results from mice treated with control or 0.4 mg/kg NPs.
**Figures 27A-27B** present bar graphs demonstrating the parasite burden in mice as measured by (**27A**) qPCR and (**27B**) microscopy.
**Figures 28A-28B** present photographs of plate assay of T. brucei cells grown of semi solid agar with (**28A**) two concentrations (Low concentration (0.2 µg/µl) represented by green dot (left) and high concentration (2 µg/µl) represented by red color (right)) of various NPs in solution or (**28B**) two concentrations (Low concentration (0.02%) represented by green dot (left) and high concentration (0.2%) represented by red color (right)) of various NPs in ointment.
**Figures 29A-29B** present (**29A**) a schematic of the ointment treatment experiments as well as photographs of the treated and untreated mice, and (**29B**) a bar graph of parasite burden as assessed by qPCR after ointment treatment.

### DETAILED DESCRIPTION OF THE INVENTION

The present invention, in some embodiments thereof, relates to a core shell particle having linked thereto a polymer and an active agent (e.g., anti-parasitic agent).

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

### The composition

The present invention, in some embodiments thereof, relates to a composition comprising at least one particle, having a core comprising metal oxide, and a shell comprising a lanthanide element, wherein the lanthanide element has linked thereto a polymer and an active agent e.g., hydrophobic active agent such as hydrophobic pentamidine wherein, the weight ratio of the active agent to the metal oxide is in the range of from 1:2 to 3:2.

The term "hydrophobic active agent" refers to an active agent having solubility in water of less than about 100 µg/mL at 25 °C and neutral pH, less than about 10 µg/mL at 25 °C and neutral pH, or less than about 5 µg/ml at 25 °C and neutral pH.

In some embodiments, the weight ratio of the active agent to the metal is in the range of from 1:2 to 3:2. As described hereinthroughout, a metal is Fe, and an active agent is pentamidine.

In some embodiments, the metal oxide is in the form of one or a plurality of nanoparticles.

In some embodiments, the metal oxide is in the form of a nanoparticle. In some embodiments, the metal oxide is in the form of a nano-rod.

In some embodiments, the metal is selected from, but is not limited to iron (ferric), aluminum, zinc, copper, manganese, cobalt, nickel, or any combination thereof. In some embodiments, an iron (III) oxide nanoparticle is in the form of gamma phase. In some embodiments, the metal oxide is magnetite (Fe₃O₄) or maghemite (gamma-Fe₂O₃; "γ-Fe₂O₃").

In some embodiments, the metal oxide particles of the present invention may be used as magnetic resonance (MR) contrast agents. These particles may yield a T2*, T2, or T1 magnetic resonance signals/signal contrast enhancement upon exposure to an external magnetic field.

In some embodiments, the lanthanide element is selected from, but is not limited to lanthanum, cerium, praseodymium, neodymium, promethium, samarium, europium, gadolinium, terbium, dysprosium, holmium, erbium, thulium, ytterbium, lutetium, or any combination thereof.

In some embodiments, the lanthanide element is in the form of an ion. In some embodiments, the lanthanide ion is a cerium ion, e.g., Ce³⁺, and Ce⁴⁺.

In some embodiments, the term "cerium" includes or can be replaced with the term "cerium phase". In one embodiment, the term "cerium" includes or can be replaced with the term "cerium (III/IV) cations" or "cerium complex". In some embodiments, the term "cerium" includes or can be replaced with the term "cerium phase (cerium (III/IV) cations)". In some embodiments, the term "cerium" includes or can be replaced with the term "ceric ammonium nitrate" [(NH₄)₂Ce(IV)(NO₃)₆); CAN].

In an embodiment of the present invention, the composition is in the form of one or more particles, wherein the particle comprises a core and a shell, wherein the core comprises a metal oxide and the shell (or the outer layer of the core) comprises cerium. In some embodiments, the ceric metal oxide particles are hydrophobic.

As described hereinthroughout, the particle is a magnetic particle. In another embodiment, the magnetic nanoparticle includes but is not limited to, particles that are magnetic, super-paramagnetic, ferromagnetic, ferromagnetic, or paramagnetic. In one embodiment, the particle can be used for contrast enhancement in X-ray, magnetic resonance imaging (MRI), or computed tomography (CT) imaging. In another embodiment, the particle can be used for bio-sensing.

In some embodiments, the term "core-shell structure" refers to a solid material, wherein the solid material is a particulate material, and wherein individual particle(s) is characterized by containing at least two different types of materials which may be distinguished from one another by their shape, by their diameter, by their composition, by their structure and/or by their placement within the particle, wherein one or more materials of a certain type are contained in the interior portion of the particle. The interior portion is designated by the term "core" or "matrix" or "inner matrix".

In some embodiments, one or more materials of a certain type which may be distinguished from the one or more materials contained in the interior portion are contained in the outer portion of the particles. The outer portion comprising the surface is designated by the terms "shell" or "coating layer" or "encapsulation layer".

In some embodiments, the metal oxide is in the center, interior, middle, or nucleus portion of the nanoparticle. In some embodiments, the metal oxide is partially or fully encapsulated or surrounded by the cerium.

In some embodiments, the shell is a continuous outer-layer. In another embodiment, the shell is non-uniform. In another embodiment, the shell is continuous, non-continuous and/or provides complete or incomplete coverage of the core. In another embodiment, the core is continuous or non-continuous.

In some embodiments, the lanthanide element is linked to one or more materials. In some embodiments, "by linked", it is meant to refer to being bound *via* an electrostatic bond, *via* a coordination bond, or *via* an ionic bond.

In another embodiment, the nucleic acid is linked to a polycationic polymer (e.g., polyethylenimine) by electrostatic bonds.

In some embodiments, the lanthanide element is coordinatively bound to a moiety of material, comprising at least one atom selected from, without being limited thereto, N, O, and S.

In some embodiments, the lanthanide element is bound to a peptide, e.g., *via* the N-terminus of the peptide. In some embodiments, the lanthanide element is bound to the peptide *via* at least one residue selected from, but not limited to: Lys, Arg, His, Glu, and Asp. In some embodiments, by "bound" (or "linked") it is meant to refer to coordinately bound.

In another embodiment, the lanthanide element is coordinatively bound to a conjugated system (e.g., Lewis base ligand conjugated to a polymer).

As used herein, the term "Lewis base" describes a substance that can donate a pair of nonbonding electrons. A Lewis base is therefore an electron-pair donor. Lewis bases are conventional amines such as ammonia and alkyl amines. Other common Lewis bases include pyridine and its derivatives or materials that comprise a moiety comprising at least one atom selected from, without being limited thereto, N, O, and S, or any combination thereof.

In some embodiments, the term "conjugated system" refers to a system of atoms covalently bonded with alternating single and multiple bonds.

As used herein, the term "polymer" describes an organic substance composed of a plurality of repeating structural units (monomeric units) covalently connected to one another.

In one embodiment, the polymer is conjugated to polysaccharides. In one embodiment, the polymer is conjugated to hydrophobic moieties. In another embodiment, the polymer is conjugated to polyethylene glycol (PEG) polymer.

In some embodiments, the polymer is in the form of a charged polymer. In one embodiment, the polymer is in the form of a polycationic polymer. In one embodiment, the polymer is in the form of a polyanionic polymer. In some embodiments, the polymer is selected from, but is not limited to: polyethylenimine (PEI), poly-L-lysine, chitosan, poly-L-arginine, dendrimeric polyamidoamine (PAMAM), polyacrylate, polyphosphate, PEG, and any derivative or combination thereof.

Particles, designated as "PEI-ceric ammonium nitrate (CAN)-*γ*-Fe₂O₃" or "PEI/Penta- CAN-*γ*-Fe₂O₃," are demonstrated herein below in the Examples section ("Penta" refers to pentamidine).

In some embodiments, the polymer is linear (e.g., as described herein below in the Examples section). In some embodiments, the polymer is branched (e.g., as described herein below in the Examples section). In some embodiments, the particles shell comprises a mixture of linear and branched PEI polymer. In some embodiments, the particles shell comprises about 25%, about 50%, or about 75% branched PEI polymer.

In some embodiments, the terms "branched", "branching", and any grammatical derivative thereof, which are used herein interchangeably, describe the replacement of a substituent atom, e.g., a hydrogen, on a monomer subunit, by another covalently bonded chain of a polymer, wherein the polymer chain has branch points that connect three or more chain segments. Controversy, a polymer architecture that prevents branching produces a "linear" polymer.

In some embodiments, the molecular weight (M_{w}) of the polymer is in the range of from 100 Da to 50 KDa. In some embodiments, the molecular weight of the polymer is in the range of from 500 Da to 60 KDa. In some embodiments, the molecular weight of the polymer is in the range of from 500 Da to 100 KDa. In some embodiments, the molecular weight of the polymer is in the range of from 20 KDa to 60 KDa. In some embodiments, the molecular weight of the polymer is in the range of from 8 KDa to 12 KDa. In some embodiments, the molecular weight of the polymer is in the range of from 600 Da to 800 Da.

As used herein, the term "weight average molecular weight" (M_{w}) generally refers to a molecular weight measurement that depends on the contributions of polymer molecules according to their sizes.

In some embodiments, the active agent is selected from, but is not limited to a therapeutic active agent (also referred to as "drug"), a diagnostic active agent, and a combination thereof. In some embodiments, the active agent is an anti-parasite agent.

In some embodiments, the therapeutic active agent is selected from, but is not limited to: pentamidine, suramin, melarsoprol, eflomithine, nifurtimox, benznidazol, fexinidazole, oxaborole, pafuramidine, doxorubicin, amphotericin B, a bioactive peptide, a bioactive nucleic acid, and any combination thereof. The active agent according to the claims is pentamidine.

In one embodiment, the peptide is in the form of a polypeptide. In some embodiments, the peptide can be synthetic, recombinant, or isolated from natural sources.

In some embodiments, the nucleic acid is selected from, but is not limited to small interfering RNA (siRNA), microRNA (miRNA), RNA interfering (RNAi), double stranded RNA (dsRNA), DNA, or any combination thereof. In another embodiment, the nucleic acid molecule is a single-stranded RNA.

In another embodiment, the active agent is a targeting ligand. In another embodiment, the targeting ligand is a molecule or a structure that provides targeting of the nanoparticle to a desired organ, tissue, cell receptor, or cell.

In some embodiments, the disclosed particle is characterized by a hydrodynamic diameter in the range of from 6 nm to 2 µm. In some embodiments, the particle is characterized by a hydrodynamic diameter in the range of from 60 nm to 2 µm. In some embodiments, the particle is characterized by a hydrodynamic diameter in the range of from 60 nm to 1.7 µm. In some embodiments, the particle is characterized by a hydrodynamic diameter in the range of from 60 nm to 800 nm. In some embodiments, the particle is characterized by a hydrodynamic diameter in the range of from 60 nm to 1.5 µm. In some embodiments, the hydrodynamic diameter is 100 to 500 nm. In some embodiments, the hydrodynamic diameter is 140 nm, 150 nm, 160 nm, 170 nm, 180 nm, 190 nm, 200 nm, 210 nm, 220 nm, 230 nm, 240 nm, 250 nm, 260 nm, 270 nm, 280 nm, 290 nm, 300 nm, 310 nm, 320 nm, 330 nm, 340 nm, 350 nm, 360 nm, 370 nm, 380 nm, 390 nm, 400 nm, 410 nm, 420 nm, 430 nm, 440 nm, 450 nm, 460 nm, 470 nm, 480 nm, 490 nm, or 500 nm, including any value and range therebetween.

In some embodiments, a plurality of the disclosed particles is characterized by a hydrodynamic diameter that varies within less than ±20%, less than ±15%, less than ±10%, or less than ±5%.

In some embodiments, the particle size distribution is characterized by a polydispersity index (PDI) of less than 0.8, less than 0.7, or less than 0.5. In some embodiments, the particle size distribution is characterized by a polydispersity index (PDI) of 0.2, 0.3, 0.4, 0.5, 0.6, or 0.7, including any value and range therebetween.

In some embodiments, the particle size distribution is characterized by a polydispersity index (PDI) of about 0.44.

As used herein, the term "polydispersity index" (PDI) refers to a measure of the distribution of the particles size.

In some embodiments, the term "hydrodynamic diameter" encompasses a size of at least one dimension, e.g., length. In some embodiments, the "diameter of the particle(s)" refers to a median size of a plurality of particles. In some embodiments, the term "hydrodynamic diameter" refers to a size as determined for a macromolecule or colloid particle in solution (e.g., aqueous solution) using any technique known in the art, e.g., dynamic light scattering (DLS) performed in an aqueous solvent.

In some embodiments, a median dry diameter of the particles ranges from 1 to 50 nm. In some embodiments, a median dry diameter of the particles ranges from 1 to 10 nm.

In some embodiments, a median dry diameter of the particles is about 1 nm, about 2 nm, about 3 nm, about 4 nm, about 5 nm, about 6 nm, about 7 nm, about 8 nm, about 9 nm, about 10 nm, about 11 nm, about 12 nm, about 13 nm, about 14 nm, about 15 nm, about 16 nm, about 17 nm, about 18 nm, about 19 nm, about 20 nm, about 21 nm, about 22 nm, about 23 nm, about 24 nm, about 25 nm, about 26 nm, about 27 nm, about 28 nm, about 29 nm, about 30 nm, about 31 nm, about 32 nm, about 33 nm, about 34 nm, about 35 nm, about 36 nm, about 37 nm, about 38 nm, about 40 nm, about 42 nm, about 44 nm, about 46 nm, about 48 nm, or 50 nm, including any value therebetween.

The term "dry diameter" is art-recognized and is used herein to refer to the physical diameter. As exemplified in the Example section that follows, the dry diameter of the polymeric particles, as prepared according to some embodiments of the invention, may be evaluated using transmission electron microscopy (TEM) or scanning electron microscopy (SEM) imaging.

The polymeric particle(s) can be generally shaped as a sphere, a rod, a cylinder, a ribbon, a sponge, and any other shape, or can be in a form of a cluster of any of these shapes, or can comprises a mixture of one or more shapes.

In some embodiments, the particle is characterized by a positive Zeta- (ζ-) potential, e.g., in an aqueous solvent.

In some embodiments, the particle is characterized by a zeta potential of at least +20 mV. In some embodiments, the particle is characterized by a zeta potential of at least about +30 mV. In some embodiments, the particle is characterized by a zeta potential in the range of from +20 mV to +70 mV. In some embodiments, the particle is characterized by a zeta potential in the range of from +20 mV to +80 mV. In some embodiments, the particle is characterized by a zeta potential in the range of +20 mV, +25 mV, +30 mV, +35 mV,+40 mV, +45 mV, or +50 mV, including any value and range therebetween.

In some embodiments, the particle is characterized by a zeta potential of about 48 mV.

In some embodiments, the composition comprises CAN-Fe₂O₃ having attached on a surface thereof PEI. In additional embodiments, the composition comprising CAN-Fe₂O₃ and PEI has further attached on a surface thereof an active agent, e.g., pentamidine. Herein "By a surface thereof", it is meant that the PEI and/or the active agent are present on at least surface of the CAN-Fe₂O₃ particles.

In some embodiments, the composition comprising CAN-Fe₂O₃ attached to PEI is characterized by infra-red (IR) spectrum with one or more unique patterns in the range of: 3520-3570 cm⁻¹, 3480-3500 cm⁻¹, 1500-1530 cm⁻¹, and 1650-1690 cm⁻¹. In some embodiments, the composition comprising CAN-Fe₂O₃ attached to pentamidine and PEI is characterized by IR spectrum with a further one or more unique patterns of: 1900-1200 cm⁻¹, 1580-1620 cm⁻¹, 1500-1530 cm⁻¹, and 830-870 cm⁻¹.

Herein, by "unique pattern" it is meant to refer to a characteristic band or a peak.

Methods for measuring IR are known in the art, e.g., Fourier-transform IR.

In some embodiments, the composition comprises PEI-CAN-γ-Fe₂O₃, wherein the PEI is present at a weight concentration of 45% to 80%, or 50% to 75%, e.g., 50%, 55%, 60%, 65%, 70%, 75%, or 80%, including any value and range therebetween. In some embodiments, the composition comprises about 67% by weight PEI. Methods for obtaining the weight concentration are known in the art, e.g., thermo-gravimetric analysis (TGA).

In another embodiment, the present invention provides a method for preventing, inhibiting or reducing microbial infection (e.g., leishmaniasis), cancer and/or neurodegenerative disorders. In some embodiments, about 70% of parasites are killed *in vivo.*

In some embodiments, and without being bound by any particular theory, the nanoparticles may be accumulated in the site (e.g., the tumor or the tissue) by an enhanced permeation and retention (EPR) effect.

In some embodiments, the composition comprises the particle and a pharmaceutically acceptable excipient. In another embodiment, the composition comprises the particle and a buffer.

### The pharmaceutical product

In some embodiments, there is provided a pharmaceutical product comprising the disclosed composition or particle, according to one or more embodiments thereof. In some embodiments, the pharmaceutical product comprises a composition of the invention and pharmaceutically acceptable excipient, adjuvant or carrier.

As used herein, the terms "administering," "administration", and like terms refer to any method which, in sound medical practice, delivers a composition containing an active agent to a subject in such a manner as to provide a therapeutic effect. One aspect of the present subject matter provides for topical administration of a therapeutically effective amount of a composition of the present subject matter to a patient in need thereof. One aspect of the present subject matter provides for intravenous administration of a therapeutically effective amount of a composition of the present subject matter to a patient in need thereof. Other suitable routes of administration can include parenteral, subcutaneous, intravenous, intramuscular, or intraperitoneal. In some embodiments, a pharmaceutical product is formulated for parenteral, mucosal, nasal, topical, intravenous or oral administration. In some embodiments, a pharmaceutical product is formulated for topical administration. In some embodiments, a pharmaceutical product is formulated for intravenous administration. In some embodiments, a pharmaceutical product is formulated for systemic administration.

The dosage administered will be dependent upon the age, health, and weight of the recipient, kind of concurrent treatment, if any, frequency of treatment, and the nature of the effect desired.

In some embodiments, the topical formulation is any one of a paste, cream, lotion, foam, gel, emulsion, ointment, and soap. In some embodiments, the topical formulation is an ointment. In some embodiments, the concentration of the composition of the invention in the formulation is at least 0.0001, 0.0002, 0.0005, 0.001, 0.002, 0.005, 0.01, 0.02, 0.05, 0.1, 0.2, 0.5, 1, 1.5, or 2 mg/ml. Each possibility represents a separate embodiment of the invention. In some embodiments, the concentration of the composition of the invention in the formulation is at most 0.0001, 0.0002, 0.0005, 0.001, 0.002, 0.005, 0.01, 0.02, 0.05, 0.1, 0.2, 0.5, 1, 1.5, or 2 mg/ml. Each possibility represents a separate embodiment of the invention. In some embodiments, the concentration of the composition of the invention in the formulation is between 0.0001- 5, 0.0002-5, 0.0005-5, 0.001-5, 0.002-5, 0.005-5, 0.01-5, 0.02-5, 0.05-5, 0.1-5, 0.2-5, 0.5-5, 1-5, 1.5-5, 2-5, 0.0001-4, 0.0002-4, 0.0005-4, 0.001-4, 0.002-4, 0.005-4, 0.01-4, 0.02-4, 0.05-4, 0.1-4, 0.2-4, 0.5-4, 1-4, 1.5-4, 2-4, 0.0001- 3, 0.0002-3, 0.0005-3, 0.001-3, 0.002-3, 0.005-3, 0.01-3, 0.02-3, 0.05-3, 0.1-3, 0.2-3, 0.5-3, 1-3, 1.5-3, 2-3, 0.0001- 2, 0.0002-2, 0.0005-2, 0.001-2, 0.002-2, 0.005-2, 0.01-2, 0.02-2, 0.05-2, 0.1-2, 0.2-2, 0.5-2, 1-2, 1.5-2, 0.0001- 1.5, 0.0002-1.5, 0.0005-1.5, 0.001-1.5, 0.002-1.5, 0.005-1.5, 0.01-1.5, 0.02-1.5, 0.05-1.5, 0.1-1.5, 0.2-1.5, 0.5-1.5, 1-1.5, 0.0001- 1, 0.0002-1, 0.0005-1, 0.001-1, 0.002-1, 0.005-1, 0.01-1, 0.02-1, 0.05-1, 0.1-1, 0.2-1, 0.5-1, 0.0001- 0.5, 0.0002-0.5, 0.0005-0.5, 0.001-0.5, 0.002-0.5, 0.005-0.5, 0.01-0.5, 0.02-0.5, 0.05-0.5, 0.1-0.5, 0.2-0.5, 0.0001- 0.2, 0.0002-0.2, 0.0005-0.2, 0.001-0.2, 0.002-0.2, 0.005-0.2, 0.01-0.2, 0.02-0.2, 0.05-0.2, or 0.1-0.2 mg/ml. Each possibility represents a separate embodiment of the invention. In some embodiments, the concentration of the composition of the invention in the formulation is at least 0.0001, 0.0002, 0.0005, 0.001, 0.002, 0.005, 0.01, 0.02, 0.05, 0.1, 0.2, 0.5, 1, 1.5, or 2%. Each possibility represents a separate embodiment of the invention. In some embodiments, the concentration of the composition of the invention in the formulation is at most 0.0001, 0.0002, 0.0005, 0.001, 0.002, 0.005, 0.01, 0.02, 0.05, 0.1, 0.2, 0.5, 1, 1.5, or 2%, by weight. Each possibility represents a separate embodiment of the invention. In some embodiments, the concentration of the composition of the invention in the formulation is between 0.0001-5, 0.0002-5, 0.0005-5, 0.001-5, 0.002-5, 0.005-5, 0.01-5, 0.02-5, 0.05-5, 0.1-5, 0.2-5, 0.5-5, 1-5, 1.5-5, 2-5, 0.0001-4, 0.0002-4, 0.0005-4, 0.001-4, 0.002-4, 0.005-4, 0.01-4, 0.02-4, 0.05-4, 0.1-4, 0.2-4, 0.5-4, 1-4, 1.5-4, 2-4, 0.0001- 3, 0.0002-3, 0.0005-3, 0.001-3, 0.002-3, 0.005-3, 0.01-3, 0.02-3, 0.05-3, 0.1-3, 0.2-3, 0.5-3, 1-3, 1.5-3, 2-3, 0.0001- 2, 0.0002-2, 0.0005-2, 0.001-2, 0.002-2, 0.005-2, 0.01-2, 0.02-2, 0.05-2, 0.1-2, 0.2-2, 0.5-2, 1-2, 1.5-2, 0.0001- 1.5, 0.0002-1.5, 0.0005-1.5, 0.001-1.5, 0.002-1.5, 0.005-1.5, 0.01-1.5, 0.02-1.5, 0.05-1.5, 0.1-1.5, 0.2-1.5, 0.5-1.5, 1-1.5, 0.0001- 1, 0.0002-1, 0.0005-1, 0.001-1, 0.002-1, 0.005-1, 0.01-1, 0.02-1, 0.05-1, 0.1-1, 0.2-1, 0.5-1, 0.0001- 0.5, 0.0002-0.5, 0.0005-0.5, 0.001-0.5, 0.002-0.5, 0.005-0.5, 0.01-0.5, 0.02-0.5, 0.05-0.5, 0.1-0.5, 0.2-0.5, 0.0001- 0.2, 0.0002-0.2, 0.0005-0.2, 0.001-0.2, 0.002-0.2, 0.005-0.2, 0.01-0.2, 0.02-0.2, 0.05-0.2, or 0.1-0.2%, by weight. Each possibility represents a separate embodiment of the invention.

In some embodiments the disclosed composition comprises the iron oxide magnetic particles nano sized as described above. In some embodiments, treatment further combines thermotherapy treatment of cutaneous diseases such as Leishmaniosis disease.

In some embodiments, the pharmaceutical product is obtained for treating a disease or disorder in a subject in need thereof. In some embodiments, the pharmaceutical product is for use in treating an infectious disease. In some embodiments, the disease or disorder is a parasitic disease. In some embodiments, the disease or disorder is a disease or disorder of the macrophage. In some embodiments, the disease or disorder is characterized by inflammation. In some embodiments, the pharmaceutical product is for use it killing an infected macrophage. In some embodiments, the infection is a parasitic infection.

In some embodiments, the pharmaceutical product is used for treatment of microbial infection. In some embodiments, the pharmaceutical product is used for killing and inhibiting microbial parasites, also referred to as: "antimicrobial activity" or "antiparasitic activity". In some embodiments, the pharmaceutical product is for use in rupturing a lysosome. In some embodiments, the pharmaceutical product is for rupturing a macrophage.

Herein "antimicrobial activity" is referring to an ability to inhibit (prevent), reduce or retard bacterial growth, fungal growth, biofilm formation or eradicate living bacterial cells, or their spores, parasites, fungal cells or viruses in a suspension or in a moist environment.

Herein "anti-parasitic activity" is referring to an ability to inhibit (prevent), reduce or retard parasitic growth, survival, or spread. Including the killing of parasite infected cells.

Herein, inhibiting or reducing or retarding the formation of load of a microorganism refers to inhibiting reducing or retarding growth of microorganisms and/or eradicating a portion or all of an existing population of microorganisms.

Thus, the disclosed composition described herein can be used both in reducing the formation of microorganisms, and in killing microorganisms in or on an article or a living tissue.

As used herein, the terms "inhibiting", "reducing", "retarding", or "preventing" refers to essentially nullifying or is reducing by at least 20 %, at least 30 %, at least 40 %, at least 50 %, at least 60 %, at least 70 %, at least 80 %, at least 90 %, including any value therebetween, of the appearance of the microorganism in a comparable situation lacking the presence disclosed nanoparticle or a composition, or a product containing same.

The microorganism can be, for example, a unicellular microorganism (prokaryotes, archaea, bacteria, eukaryotes, protists, fungi, algae, euglena, protozoan, dinoflagellates, apicomplexan, trypanosomes, amoebae and the likes), or a multicellular microorganism.

In some embodiments, the subject is a mammal. In some embodiments, the subject is a human. In some embodiments, the subject suffers from a disease or disorder. In some embodiments, the subject is at risk of developing a disease or disorder.

In some embodiments, the parasite is a leishmanial parasite. In some embodiments, the parasite is selected from *Trypanosoma brucei, leishmania donovani, leishmania tropica* and *leishmania major.* In some embodiments, the parasite is a skin parasite. In some embodiments, the parasite is a systemic parasite. In some embodiments, the parasite is a parasite that can be phagocytosed by macrophages. In some embodiments, the parasite is a parasite that is targeted by macrophages. In some embodiments, the parasite is a parasite that infects macrophages. In some embodiments, the parasite is in the amastigote stage. In some embodiments, the parasite is in the promastigote stage. In some embodiments, the parasite is in the amastigote or promastigote stage.

In some embodiments, the pharmaceutical product is used for killing and inhibiting leishmanial parasites. In some embodiments, the pharmaceutical product is used for killing and inhibiting parasites in the amastigote phase. In some embodiments, the pharmaceutical product is used for killing and inhibiting parasites in the promastigote phase. In some embodiments, the parasite is selected from, but is not limited to: *Trypanosoma brucei, leishmania donovani, leishmania tropica* and *leishmania major,* and any combination thereof.

In some embodiments, the product is in the form of paste, cream, lotion, foam, gel, emulsion, an ointment, cream and soap, without being limited thereto.

In some embodiments, a "pharmaceutical product" refers to a preparation of one or more of the active ingredients described herein with other chemical components such as physiologically suitable carriers and excipients. The purpose of a pharmaceutical product is to facilitate administration of a compound to an organism.

In one embodiment, "active ingredient" refers to the NPs of the invention, which is accountable for the biological effect.

In one embodiment, the present invention provides combined preparations. In one embodiment, "a combined preparation" defines especially a "kit of parts" in the sense that the combination partners as defined above can be dosed independently or by use of different fixed combinations with distinguished amounts of the combination partners i.e., simultaneously, concurrently, separately or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In one embodiment, the carrier is a physiologically acceptable carrier. In one embodiment, the phrases "physiologically acceptable carrier" and "pharmaceutically acceptable carrier" which be interchangeably used refer to a carrier or a diluent that does not cause significant irritation to an organism and does not abrogate the biological activity and properties of the administered compound. An adjuvant is included under these phrases. In one embodiment, "excipient" refers to an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

As used herein, the term "carrier," "excipient," or "adjuvant" refers to any component of a pharmaceutical composition that is not the active agent. As used herein, the term "pharmaceutically acceptable carrier" refers to non-toxic, inert solid, semi-solid liquid filler, diluent, encapsulating material, formulation auxiliary of any type, or simply a sterile aqueous medium, such as saline. Some examples of the materials that can serve as pharmaceutically acceptable carriers are sugars, such as lactose, glucose and sucrose, starches such as corn starch and potato starch, cellulose and its derivatives such as sodium carboxymethyl cellulose, ethyl cellulose and cellulose acetate; powdered tragacanth; malt, gelatin, talc; excipients such as cocoa butter and suppository waxes; oils such as peanut oil, cottonseed oil, safflower oil, sesame oil, olive oil, corn oil and soybean oil; glycols, such as propylene glycol, polyols such as glycerin, sorbitol, mannitol and polyethylene glycol; esters such as ethyl oleate and ethyl laurate, agar; buffering agents such as magnesium hydroxide and aluminum hydroxide; alginic acid; pyrogen-free water; isotonic saline, Ringer's solution; ethyl alcohol and phosphate buffer solutions, as well as other non-toxic compatible substances used in pharmaceutical formulations. Some substances which can serve as a carrier herein include sugar, starch, cellulose and its derivatives, powered tragacanth, malt, gelatin, talc, stearic acid, magnesium stearate, calcium sulfate, vegetable oils, polyols, alginic acid, pyrogen-free water, isotonic saline, phosphate buffer solutions, cocoa butter (suppository base), emulsifier as well as other non-toxic pharmaceutically compatible substances used in other pharmaceutical formulations. Wetting agents and lubricants such as sodium lauryl sulfate, as well as coloring agents, flavoring agents, excipients, stabilizers, antioxidants, and preservatives may also be present. Any non-toxic, inert, and effective carrier may be used to formulate the compositions contemplated herein. Suitable pharmaceutically acceptable carriers, excipients, and diluents in this regard are well known to those of skill in the art, such as those described in The Merck Index, Thirteenth Edition, Budavari et al., Eds., Merck & Co., Inc., Rahway, N.J. (2001); the CTFA (Cosmetic, Toiletry, and Fragrance Association) International Cosmetic Ingredient Dictionary and Handbook, Tenth Edition (2004); and the "Inactive Ingredient Guide," U.S. Food and Drug Administration (FDA) Center for Drug Evaluation and Research (CDER) Office of Management. Examples of pharmaceutically acceptable excipients, carriers and diluents useful in the present compositions include distilled water, physiological saline, Ringer's solution, dextrose solution, Hank's solution, and DMSO. These additional inactive components, as well as effective formulations and administration procedures, are well known in the art and are described in standard textbooks, such as Goodman and Gillman's: The Pharmacological Bases of Therapeutics, 8th Ed., Gilman et al. Eds. Pergamon Press (1990); Remington's Pharmaceutical Sciences, 18th Ed., Mack Publishing Co., Easton, Pa. (1990); and Remington: The Science and Practice of Pharmacy, 21st Ed., Lippincott Williams & Wilkins, Philadelphia, Pa., (2005). The presently described composition may also be contained in artificially created structures such as liposomes, ISCOMS, slow-releasing particles, and other vehicles which increase the half-life of the peptides or polypeptides in serum. Liposomes include emulsions, foams, micelles, insoluble monolayers, liquid crystals, phospholipid dispersions, lamellar layers and the like. Liposomes for use with the presently described peptides are formed from standard vesicle-forming lipids which generally include neutral and negatively charged phospholipids and a sterol, such as cholesterol. The selection of lipids is generally determined by considerations such as liposome size and stability in the blood. A variety of methods are available for preparing liposomes as reviewed, for example, by Coligan, J. E. et al, Current Protocols in Protein Science, 1999, John Wiley & Sons, Inc., New York, and see also U.S. Pat. Nos. 4,235,871, 4,501,728, 4,837,028, and 5,019,369.

The carrier may comprise, in total, from about 0.1% to about 99.99999% by weight of the pharmaceutical compositions presented herein.

Techniques for formulation and administration of drugs are found in "Remington's Pharmaceutical Sciences," Mack Publishing Co., Easton, PA, latest edition.

### The process

According to an aspect of some embodiments of the present invention, there is provided a process for obtaining a composition comprising at least one particle, having a core comprising metal oxide, and a shell comprising a lanthanide element, wherein the lanthanide element is linked to a polymer and an active agent, the process comprising the steps of:
(c) mixing a solvent, an ion of a lanthanide element, and metal oxide particles, thereby obtaining a suspension;
(d) adding the polymer and the active agent to the suspension.

In some embodiments, the term "suspension" refers to a heterogeneous mixture containing solid particles that do not dissolve but get suspended throughout the bulk of the solvent.

In some embodiments, the solvent is an organic solvent. In some embodiments, the solvent is an aprotic solvent. In some embodiments, the solvent is a mixture of aprotic and protic solvents.

In some embodiments, the ion of the lanthanide element is cerium.

In some embodiments, the solvent is selected from, but is not limited to: dimethylformamide (DMF), acetonitrile (ACN), water, and any combination thereof. One skilled in the art is capable to determine the suitable solvent.

In some embodiments, the mixing step is performed with DMF and ACN solvents. In some embodiments, the ratio of DMF to ACN solvents is 8:4, respectively. In some embodiments, the ratio of DMF to ACN solvents is in the range of from 10:1 to 4:1, respectively. In some embodiments, the ratio of DMF to ACN is in the range of from 5:1 to 4:1, respectively. In some embodiments, the ratio of DMF to ACN is in the range of from 5:1 to 3:1, respectively. In some embodiments, the ratio of DMF to ACN is in the range of from 10:1 to 1:2, respectively.

In some embodiments, the term "protic solvent" refers to a solvent that contains labile H⁺, wherein such solvents can donate protons (H⁺) to reagents. Conversely, "aprotic solvents" cannot donate hydrogen.

In some embodiments, the mixing step is performed under ultrasonic agitation.

In some embodiments, the adding of the polymer and the organic compound is performed simultaneously. In some embodiments, the mixing step is performed under ultrasonic agitation. In some embodiments, the step of adding the polymer and the organic compound is performed under shaking.

In some embodiments, the mixing step is performed at a temperature in the range of 5°C to 15°C. In some embodiments, the mixing step is performed at a temperature in the range of 20°C to 30°C. In some embodiments, the mixing step is performed at a temperature in the range of 15°C to 20°C. In some embodiments, the mixing step is performed at a temperature in the range of 5°C to 30°C.

In some embodiments, the particles are obtained after 2 to 6 hours from the mixing step. In some embodiments, the particles are obtained after 45 to 55 hours from the mixing step. In some embodiments, the particles are obtained after 20 to 30 hours from the mixing step.

As described herein, in some embodiments, the particle comprises PEI-CAN-*γ*-Fe₂O₃ NP(s) attached to an active agent. In some embodiments of the process, the PEI-CAN-*γ-*Fe₂O₃ NP is prepared and thereafter the active agent (e.g., pentamidine) is directly linked onto PEI-CAN-*γ*-Fe₂O₃ NP ("process A"). In additional embodiments of the process, the CAN-maghemite NP is first grafted with pentamidine, and thereafter linked with the PEI polymer ("process B"). In additional embodiments of the process, the CAN-maghemite NP is grafted simultaneously with both mixed PEI polymer and the active agent (e.g., pentamidine) ("process C"). In some embodiments, the product obtained by process C is characterized by a superior anti-parasite activity.

The basic concepts of design of experiments (DoE) is a procedure for planning and predicting experiments so that the data obtained can be analyzed using few experiments while still obtaining valid and objective conclusions.

In some embodiments, in the disclosed process, the data resulting from performing the selected DoE are fed into an algorithm which calculates efficient conditions for optimization when using e.g., hybrid mixed various polycationic PEI-type polymers (e.g., linear, branched types) for efficient of anti-parasitic properties.

The term "efficient" is understood to mean herein suitable, optimal or useful conditions for a predetermined purpose. In this context, "optimal" means the conditions which provide the highest anti parasitic activity of the disclosed composition, as affected in *vivo* and/or *in vitro.*

In some embodiments of DoE optimization process are described herein below under the Examples section.

### General

As used herein the term "about" refers to ± 10 %.

The terms "comprises", "comprising", "includes", "including", "having" and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein, the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples which, together with the above descriptions, illustrate the invention in a non-limiting fashion.

### EXAMPLE 1

### Fabrication and characterization of PEI-CAN-γ--Fe₂O₃ NPs

### Materials and Methods

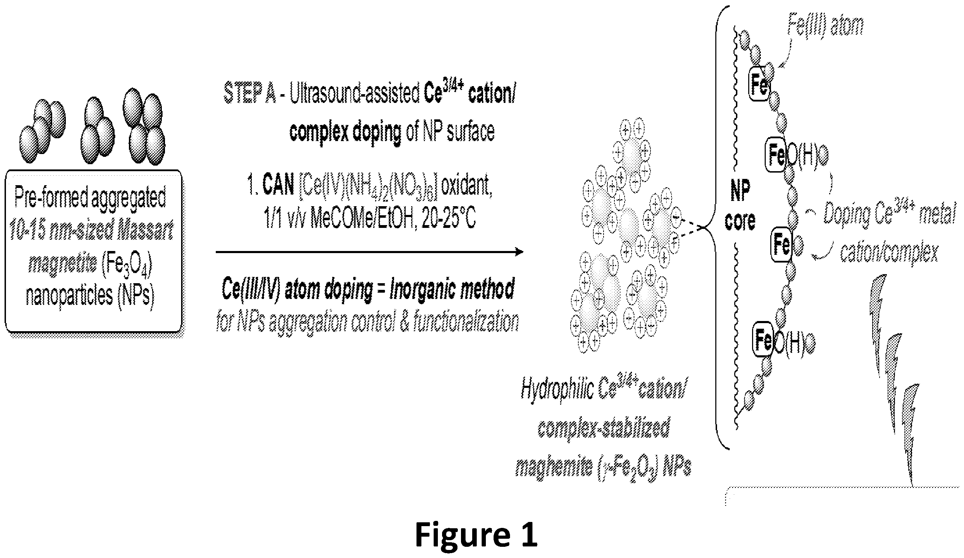

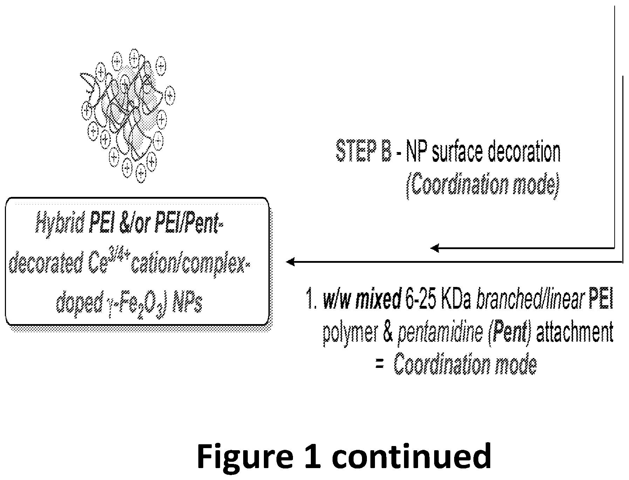

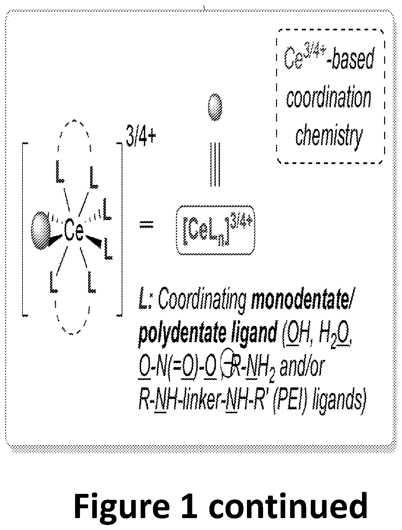

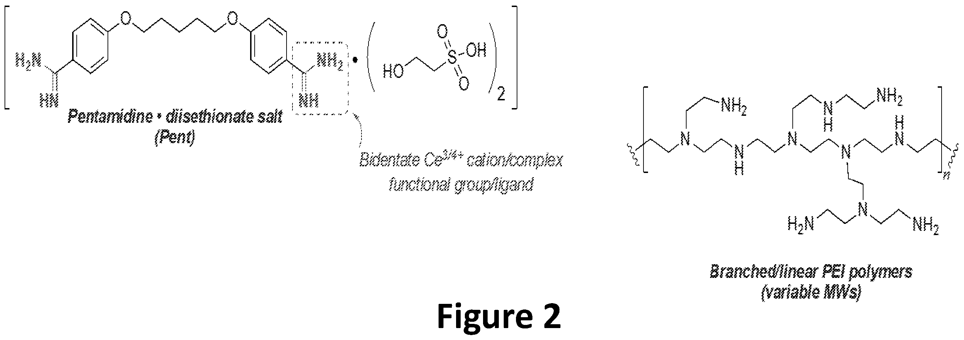

In some procedures, ceric ammonium nitrate (CAN)-*γ-*-Fe₂O₃ nanoparticles (NPs) were fabricated by high-power ultrasonication (Sonics, Vibra cell, 750 W, power modulator setup at 25% with a Titanium horn for 30 min, 0°C, dry inert argon atmosphere), and then were coated by branched or linear polyethylenimine (PEI; **Figure 2** illustrates its molecular structure) at various molecular weights including 0.6-0.8, 10 and 25 KDa under shaking conditions overnight at room temperature. As shown in **Figure 1**, Lewis acid Ce^{3/4+} cation/complex was used to coordinate with PEI polymeric species, in order to obtain several products:
1. ***_{con}*PEI₂₅-CAN-*γ*-Fe₂O₃ NPs** were fabricated from a 25 KDa branched PEI polymer.
2. ***_{con}*PEI_{0.8}-CAN-*γ*-Fe₂O₃ NPs** were fabricated from a 0.6-0.8 KDa branched PEI.
3. ***b*PEI_{0.8-25}-CAN-*γ*-Fe₂O₃ NPs** were fabricated from 50% 0.6-0.8 KDa and 50% 25 KDa of branched PEI (*b*PEI).
4. ***b*PEI0.8₇₅-25₂₅-CAN-*γ*-Fe₂O₃ NPs** were fabricated from 75% 0.6-0.8 KDa and 25% 25 KDa of branched PEI.
*5.* ***Linear* PEI₁₀-CAN-*γ*-Fe₂O₃ NPs** were fabricated from a 10 KDa linear PEI.
6. ***Linear* PEI₁₀₋₂₅-CAN-*γ*-Fe₂O₃ NPs** were fabricated from 50% of 10 KDa linear PEI and 50% of 25 KDa branched PEI.
7. ***Linear* PEI 10₇₅-25₂₅-CAN-*γ*-Fe₂O₃ NPs** were fabricated from 75% of 10 KDa linear PEI and 25% of 25 KDa branched PEI.

These NPs were characterized by dynamic light scattering (DLS) to measure hydrodynamic diameter, polydispersity index (PDI), ζ potential and Fe amounts were measured by inductively coupled plasma (ICP).

***In vitro* studies:** *Trypanosoma brucei* procyclic cells (10⁶) were incubated for 24 h with *_{con}*PEI₂₅-CAN-*γ*-Fe₂O₃ NPs or CAN-*γ*-Fe₂O₃ NPs at the indicated concentration based on (Fe) and live cells were counted. Furthermore, the lysosome was stained with lysotracker (Cys3) and the images were taken using Nikon Eclipse 90i microscope in X63 magnification. In addition, cell viability of various strains of leishmania as: *leishmania donovani, L. tropica* and *L. major* were examined for cell viability after treatment with *_{con}*PEI₂₅-CAN-*γ*-Fe₂O₃ NPs at 0.25 and 0.375 µg/ml concentrations.

The toxicity of *_{con}*PEI₂₅-CAN-*γ*-Fe₂O₃ NPs was examined by MTT assay in human macrophage cells at concentrations of from 0.5 to 2 µg/ml.

### Results

The characteristics of various PEI-CAN-γ-Fe₂O₃ NPs are summarized in **Table 1.**

**Table 1**

| **PEI-CAN-γ Fe₂O₃ NPs** | **Batch** | **Diameter (nm)** | **PDI** | **ζ potential (mV)** | **Fe concentration (mg/ml)** |
|---|---|---|---|---|---|
| ***_{con}*PEI_{0.8}**-**CAN-*γ*-Fe₂O₃** | SO08 | 3,548 | 0.337 | 23.8 | 0.842 |
| ***b*PEI_{0.8- 25}/CAN-*γ-*Fe₂O₃** | SO0825 | 9,237 | 0.289 | 24.6 | 0.294 |
| ***b*PEI0.8₇₅**-**25₂₅/CAN-*γ-*Fe₂O₃** | SO08₇₅25₂₅ | 1,860 | 1.000 | 29.1 | 0.935 |
| ***Linear* PEI₁₀/CAN-*γ-*Fe₂O₃** | SOlO | 335 | 0.466 | 43.2 | 0.055 |
| ***Linear* PEI₁₀**-**₂₅/CAN-*γ-*Fe₂O₃** | SO1025 | 347 | 0.394 | 41.6 | 0.171 |
| ***Linear* PEI 10₇₅**-**25₂₅/CAN-*γ-*Fe₂O₃** | SO10₇₅25₂₅ | 446 | 0.474 | 33.6 | 0.100 |

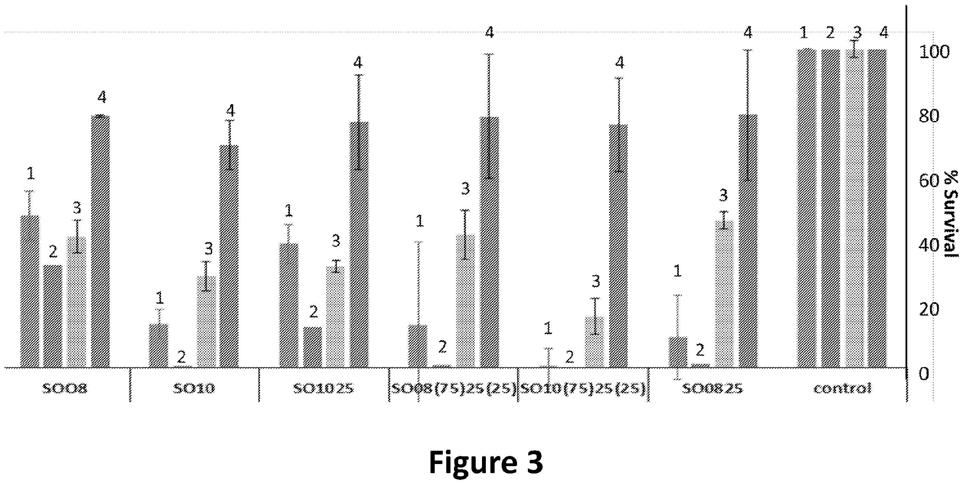

**Figure 3** demonstrates the viability of promastigotes, amastigotes and monocytes cells after treatment with the NPs listed in **Table 1.** All the compounds had stronger effects on promastigotes than amastigotes when dosed at equal concentrations. All the compounds also had similar effects on monocytes as measured by MTT assay; monocytes were significantly less sensitive to these compounds.

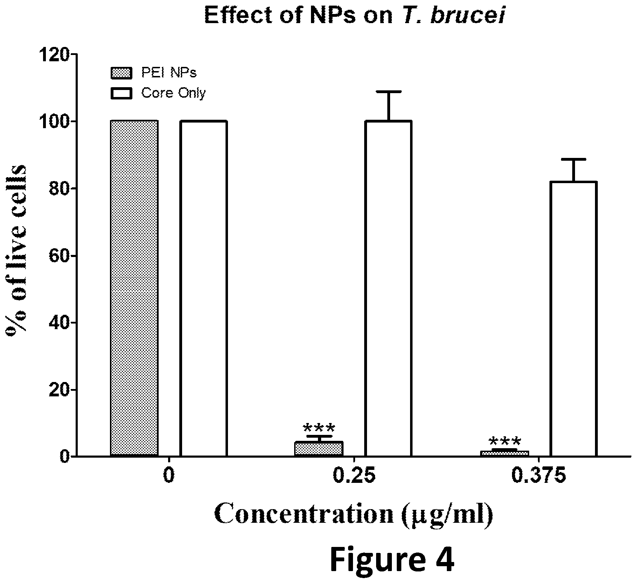

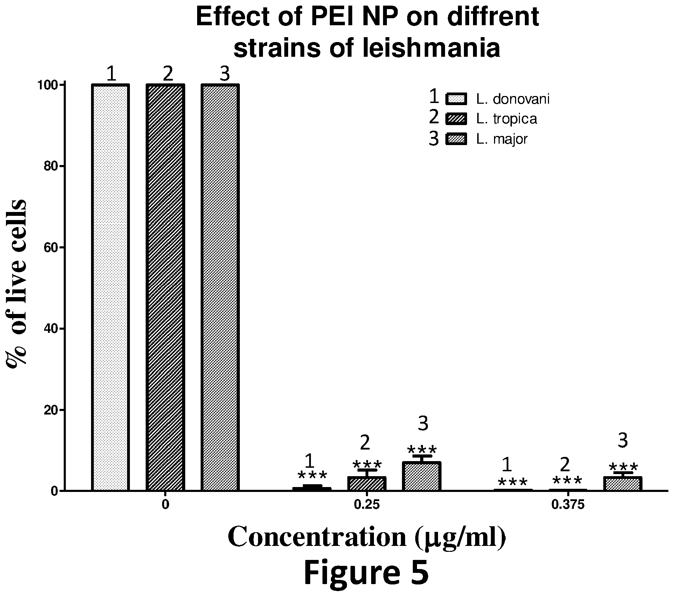

**Figures 4-5** show that *_{con}*PEI₂₅-CAN-γ-Fe₂O₃ NPs killed *T. brucei* parasites for all leishmanial strains tested. *Leishmania* promastigote cells from the indicated different strains (2 × 10⁶, Fig. 4) were also incubated for 24 hrs with the same *conPEI₂₅-CAN-γ-Fe₂O₃ NPs* at the indicated concentration based on (Fe) and live cells were then counted. **Figure 4** indicates that the PEI polymer is an essential factor for parasite elimination as the core molecule (CAN-γ-Fe₂O₃) had almost no effect on survival. As seen in **Figure 5****,** all three testes strains were susceptible to the molecule, although to slightly varying degrees.

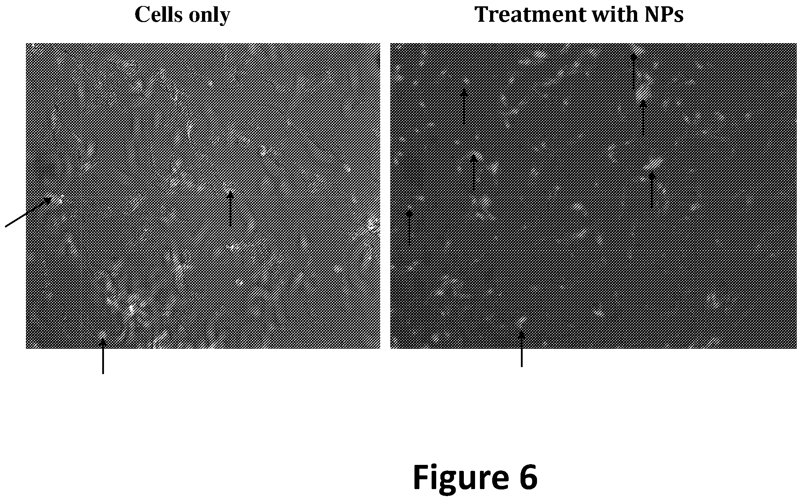

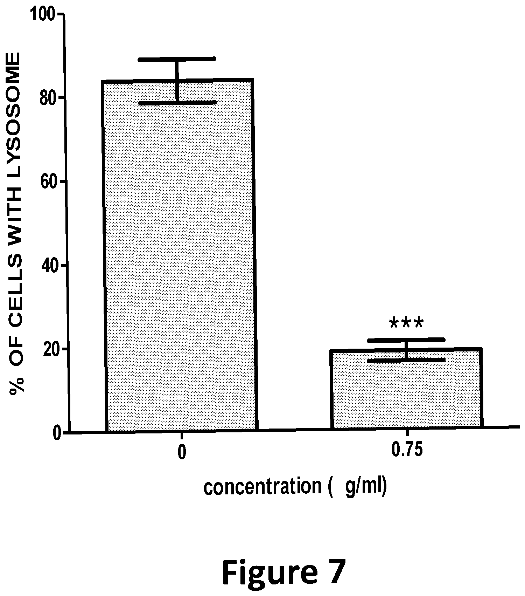

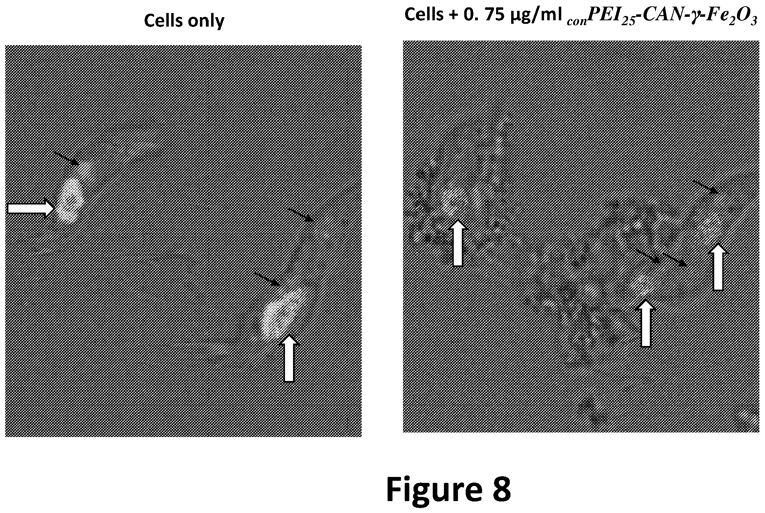

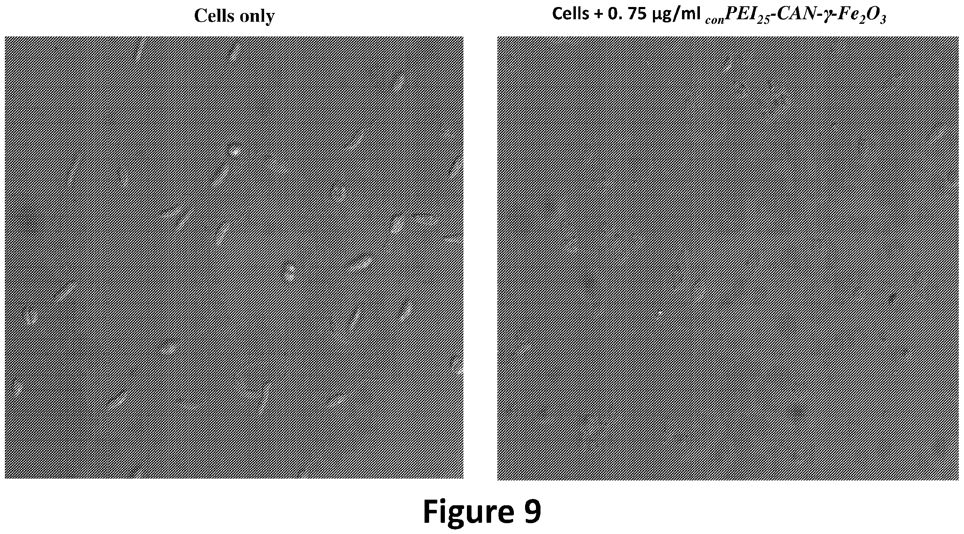

To further investigate the mechanism by which *_{con}*PEI₂₅-CAN-γ-Fe₂O₃ NPs kill infected cells, *T. brucei* cells (10⁶) were treated with the molecule at a concentration of 0.375 µg/ml for 5 minutes. Cells were washed with PBS and the lysosome was stained with lysotracker (Cys3). The images were *taken* using the Nikon Eclipse 90i microscope in X63 magnification. **Figures 6-9** illustrate the lysosome bursting mechanism caused by treatment with *_{con}*PEI₂₅-CAN-γ-Fe₂O₃ NPs. As can be seen in **Figure 6****,** and is quantified in **Figure 7** treatment with *_{con}*PEI₂₅-CAN-γ-Fe₂O₃ resulted in an extreme expansion of the lysosome, with 4 times the number of cells staining positive to lysotracker. **Figure 8** shows treated cells in the process of rupturing due to the treatment. These images are from *T. brucei* procyclic cells expressing histone-YFP treated with the NPs at a concentration of 0.75µg/ml. The pictures were taken by live imaging (a picture every five minutes) using Zeiss Observer microscope Z1 at magnification of X40. A time lapse video of the untreated cells is available at https://youtu.be/qI_oMY4rg9A and a video of the treated cell bursting is available at https://youtu.be/EUA_19guNcQ. **Figure 9** shows L. donovani promastigote cells treated with 0.75µg/ml NPs for 5 minutes, washed with PBS and stained with lysotracker. The corresponding images were taken using Nikon Eclipse 90i in X63 magnification. Burst, dead and dying cells are only seen after treatment.

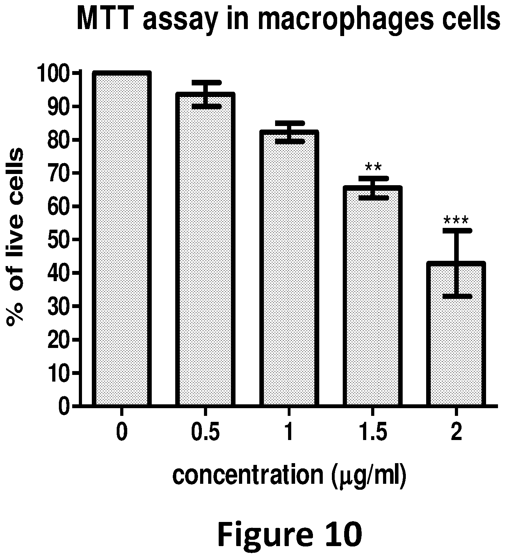

To examine the toxicity of the NPs on human cells, macrophages were treated with *_{con}*PEI₂₅-CAN-γ-Fe₂O₃. THP1 monocytes were differentiated into macrophages by PMA (Phorbol 12-Myristate 13-acetate) treatment and then incubated with the NPs at various concentrations. The cells were then incubated with MTT reagent for 20 minutes and cell viability was assessed (**Fig. 10**). Analysis was done using a one-way ANOVA test with **P<0.005 ***P value <0.0001.

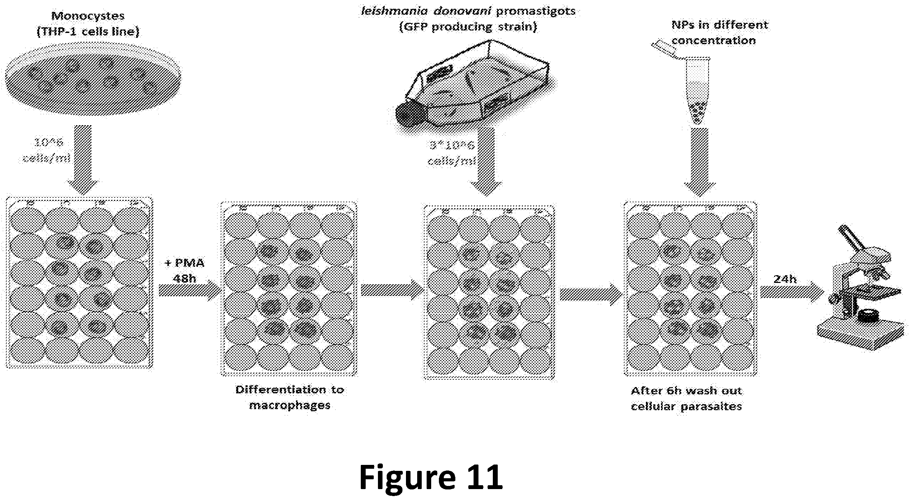

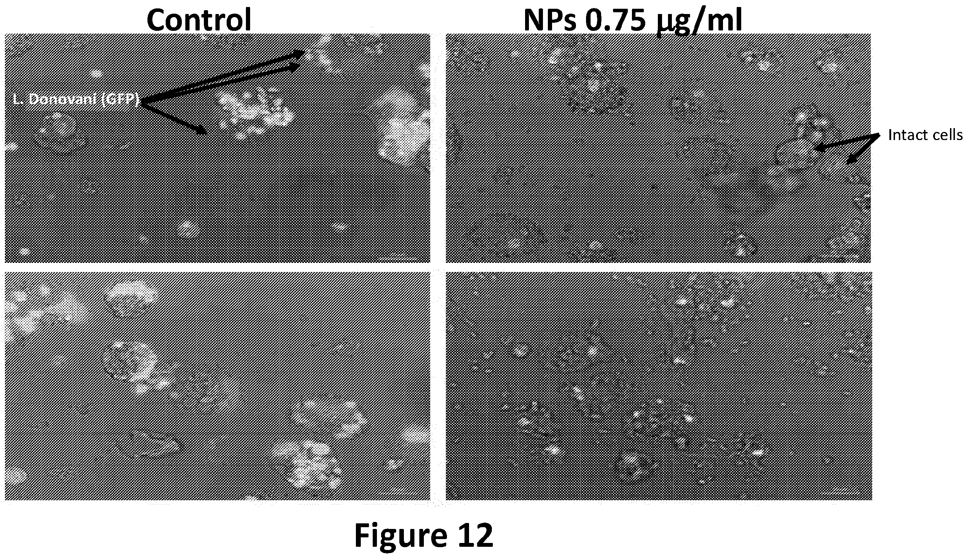

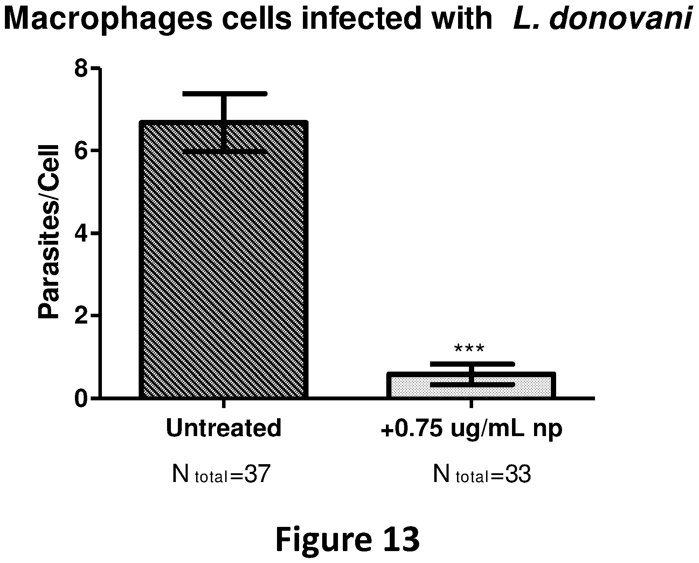

Next, the ability of *_{con}*PEI₂₅-CAN-γ-Fe₂O₃ to kill human stage parasites, i.e. the amastigote stage was examined. Human THP1 cells were plates, treated with PMS to induce differentiation to macrophages and then infected with a GFP positive strain of L. donovani promastigots (**Fig. 11**). *_{con}*PEI₂₅-CAN-γ-Fe₂O₃ at a concentration of 0.75 µg/ml were incubated with the infected macrophages and after 24 hours cell survival was assayed (**Fig. 11-12**). As can be seen in **Figure 12** control untreated cells were highly infected and thus GFP positive, whereas treatment irradicated the majority of infected cells while non-infected cells remained intact. These results are quantified in **Figure 13****.**

### EXAMPLE 2

### Optimal fabrication and characterization of _{con}PEI₂₅/Pentamidine-CAN-γ-Fe₂O₃ NPs

There are three different methods to fabricate *_{con}*PEI₂₅/Pentamidine-CAN-γ-Fe₂O₃ NPs: (i) binding of pentamidine onto *_{con}*PEI₂₅-CAN-γ-Fe₂O₃ NPs; (ii) binding of pentamidine onto CAN-γ-Fe₂O₃ NPs and then addition of PEI polymer; (iii) a simultaneous addition of both PEI polymer and pentamidine drug.

A statistically designed experiment (DoE) for optimized engineering of NPs was performed in order to identify a maximized reproducible level of parasite death while preserving robust/reproducible NPs physico-chemical characteristics and importantly, a minimal toxicity to the host. This DoE study enables to modify more than one factor/reaction condition at a time for fabrication optimization even when involving numerous influential factors. The investigated conditions are presented in **Table 2.**

In exemplary procedures, *_{con}*PEI₂₅-CAN-γ-Fe₂O₃ NPs were produced with various pentamidine concentrations as detailed in **Table 2.** THP1 derived macrophages were again employed and the cells were examined by FACS to observe DNA fragmentation (Sub-G1). These NP's ability to kill macrophages cells and the parasites either at promastigote or amastigote stages was examined at a concentration of 0.5 µg/ml.

**Table 2**

| **Sample** | **Solvent** | **Pentamidine/Fe ratio** | **Temperature (°C)** | **Reaction time (hours)** |
|---|---|---|---|---|
| **1** | DMF | 0.75 | 10 | 4 |
| **2** | DMF/ACN 8/4 | 0.25 | 26 | 48 |
| **3** | DMF | 0.25 | 10 | 4 |
| **4** | DMF | 0.75 | 10 | 48 |
| **5** | DMF/ACN 8/4 | 0.75 | 26 | 48 |
| **6** | DMF | 0.25 | 10 | 48 |
| **7** | DMF/ACN 8/4 | 0.75 | 10 | 4 |
| **8** | DMF | 0.25 | 26 | 48 |
| **9** | DMF | 0.5 | 18 | 26 |
| **10** | DMF/ACN 8/4 | 0.75 | 26 | 4 |
| **11** | DMF | 0.25 | 26 | 4 |
| **12** | DMF/ACN 8/4 | 0.5 | 18 | 26 |
| **13** | DMF/ACN 8/4 | 0.25 | 10 | 4 |
| **14** | DMF | 0.75 | 26 | 4 |
| **15** | DMF | 0.75 | 26 | 48 |
| **16** | DMF/ACN 8/4 | 0.25 | 26 | 4 |
| **17** | DMF/ACN 8/4 | 0.25 | 10 | 48 |
| **18** | DMF/ACN 8/4 | 0.75 | 10 | 48 |

A DoE four factor-two level full factorial experimental array was designed and analyzed using a MINITAB^{®} 17 DoE software (version 17.2, Minitab Inc. Ltd). This design also included one factor replicate (one repetition of experiments) and two center points.

The optimized results were plotted by two-dimensional contour similar to topographic maps. These plots show two reaction factors on axes X and Y, while the chosen response is color-encoded at Z axis. An interaction plot basically reveals if there is an effective interaction between two factors or reaction conditions for a given response. When the two considered graphs are either super-imposed or parallel, it means that there is no interaction between these two factors. On the contrary, line intersection indicates a strong interaction between these two considered factors.

In order to calculate an optimal set of reaction conditions, the MINITAB software optimizer tool was used, and the corresponding importance of DoE-responses defined. The most important responses were set to be the biological assays for amastigote cells (set to be less than 25%) as well as the apoptosis assay in host macrophages (NPs toxicity, set to be above 85%). The biological promastigote assays and PDI were also optimized but with less importance.

The optimal batch was tested using HPLC system ELITE LaChrom (Hitachi Systems) by fluorescence detection of pentamidine (Excitation: 270 nm, Emission: 345 nm), in order to quantify pentamidine amount attached onto the NPs surface with PEI polymer. Chromatography was performed under isocratic conditions using a mixture of 25 mM potassium phosphate pH 3.2 and acetonitrile (ACN) in a ratio of 88:12, respectively, on LIChrospher^{®} 100 RP-18e (5µm) 125 × 4 mm ID at flow rate of 1 mL/min. Furthermore, a standard curve was developed using pentamidine salt at concentrations of 0.25, 0.5, 1.0, 2.5, 5.0 and 10 µg/mL.

Moreover, the optimal batch was characterized by FTIR, thermogravimetric analyses (TGA) for measuring the PEI amounts, MTT assay with various concentrations of NPs (0.25-1 µg/ml), toxicity assay in monocytes cells after treatment with 0.5 µg/ml, and cell viability of amastigotes and promastigotes.

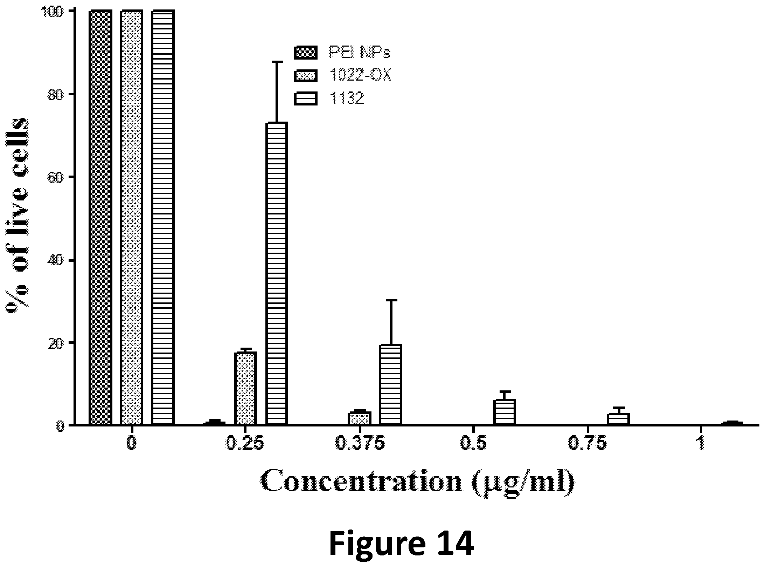

***In vitro* studies:** The new pentamidine-grafted NPs were almost as effective as the original *_{con}*PEI₂₅-CAN-γ-Fe₂O₃ NPs at killing L. donovani promastigote stage after 24 hours (**Fig. 14**). At a concentration of 0.5 µg/ml there was close to 90% cell death. The production method was next optimized for enhanced parasite killing.

In order to study how the reaction factors (solvent, pentamidine/Fe ratio, temperature and reaction time) interact with each other and how such interaction will influence on final global DoE-optimization results, we focused on seven quantified responses (reaction conditions outputs), *i.e.,* (i) the average DLS hydrodynamic size of resulting surface-engineered NPs with polydispersity indexing (PDI), (ii) their ξ potential values, (iii) the amount of pentamidine attached to the NPs, measured by HPLC and calculated to a final Pentamidine/Fe w/w ratio, (iv) the MTT toxicity assay (% of life cells, after 24h, C = 0.5 µg/mL) (v-vi) and both Amastigote and Promastigote parasites cell viability (24h, C = 0.5 µg/mL) (**Table 3**).

First, response analyses that characterized the experimental array under investigation indicated that both the DLS values (from 113 to 406 nm), PDI (from 0.35 to 0.69) and ζ potential values (from +23.0 to +39. mV) obtained for all the 18 DoE-engineered NPs fabricated with pentamidine incorporation (*_{con}*PEI₂₅-Penta-CAN-*γ*-Fe₂O₃ NPs, **Table 3**) were mostly higher than the values obtained for basic non-Penta-modified *_{con}*PEI₂₅-CAN-*γ-*Fe₂O₃ NPs (106.2 nm, 0.329 and +25.3 mV respectively).

Without being bound by any particular theory, it is assumed that the measured higher ζ potential values are likely due to a coordinative attachment of pentamidine (bidentate amidine function) onto the Ce^{3/4+} cations on the NPs surface. This coordinative binding is actually "blocking" some of the Ce^{3/4+} sites, forcing the coordinated PEI polymer phase to bind with less numerous involved primary amine groups (-NH₂) which causes NPs ζ potential values increases as observed

In addition, the mother liquor of the cleaning phase of the optimal batch was tested using HPLC system ELITE LaChrom (Hitachi Systems), in order to quantify how much pentamidine was actually coordinatively attached onto the NPs surface within the PEI phase. HPLC was accomplished using Fluorescence detection of pentamidine (Excitation: 270 nm, Emission: 345 nm). Chromatography was performed under isocratic conditions using a 25mM potassium phosphate pH 3.2-acetonitrile mixture in a 88:12 ratio on LIChrospher^{®} 100 RP-18e (5µm) 125 × 4 mm ID at the flow rate of 1 mL/min. ²³ In order to quantify how much pentamidine was actually attached to the NPs surface, a standard curve was developed using pentamidine salt at concentrations of 0.25, 0.5, 1.0, 2.5, 5.0 and 10 µg/mL. This measurement disclosed a range of 0.01-0.69 pentamidine/Fe W/W.

**Table 3: DoE-experimental array with the corresponding NPs-relating responses/outputs.**

| **Run Order** | **DLS [nm]** | **PDI** | **ζ [mV]** | **Pentamidine /Fe w/w final ratio** | **MTT Test** | **Amastigote at c=0.5 [%]** | **Promastigote at c=0.5[%]** |
|---|---|---|---|---|---|---|---|
| 1 | 115.0 | 0.35 | 28.7 | 0.09 | 91.00 | 23.00 | 0.00 |
| 2 | 175.4 | 0.56 | 26.7 | 0.69 | 83.08 | 45.31 | 0.00 |
| 3 | 135.3 | 0.39 | 29.6 | 0.17 | 78.72 | 35.53 | 0.00 |
| 4 | 211.0 | 0.42 | 35.4 | 0.01 | 76.69 | 42.54 | 0.00 |
| 5 | 325.0 | 0.58 | 34.9 | 0.31 | 84.90 | 52.70 | 0.00 |
| 6 | 112.9 | 0.36 | 37.5 | 0.25 | 68.14 | 33.42 | 0.00 |
| 7 | 309.4 | 0.69 | 39.4 | 0.17 | 79.40 | 49.62 | 0.00 |
| 8 | 151.4 | 0.55 | 30.8 | 0.15 | 92.80 | 33.47 | 0.00 |
| 9 | 256.0 | 0.52 | 27.1 | 0.06 | 82.23 | 24.42 | 0.00 |
| 10 | 406.0 | 0.60 | 29.8 | 0.27 | 82.88 | 31.62 | 0.11 |
| 11 | 156.7 | 0.40 | 31.7 | 0.05 | 72.54 | 26.86 | 0.05 |
| 12 | 239.4 | 0.56 | 35.4 | 0.17 | 74.72 | 40.23 | 0.00 |
| 13 | 130.2 | 0.39 | 39.0 | 0.25 | 73.71 | 29.75 | 0.00 |
| 14 | 235.9 | 0.49 | 30.0 | 0.08 | 87.25 | 51.93 | 0.00 |
| 15 | 379.0 | 0.63 | 23.0 | 0.10 | 79.83 | 40.62 | 0.00 |
| 16 | 141.7 | 0.41 | 31.6 | 0.12 | 73.90 | 31.81 | 0.00 |
| 17 | 120.2 | 0.37 | 36.3 | 0.13 | 78.98 | 26.67 | 0.00 |
| 18 | 199.4 | 0.46 | 34.3 | 0.08 | 75.63 | 38.23 | 1.16 |

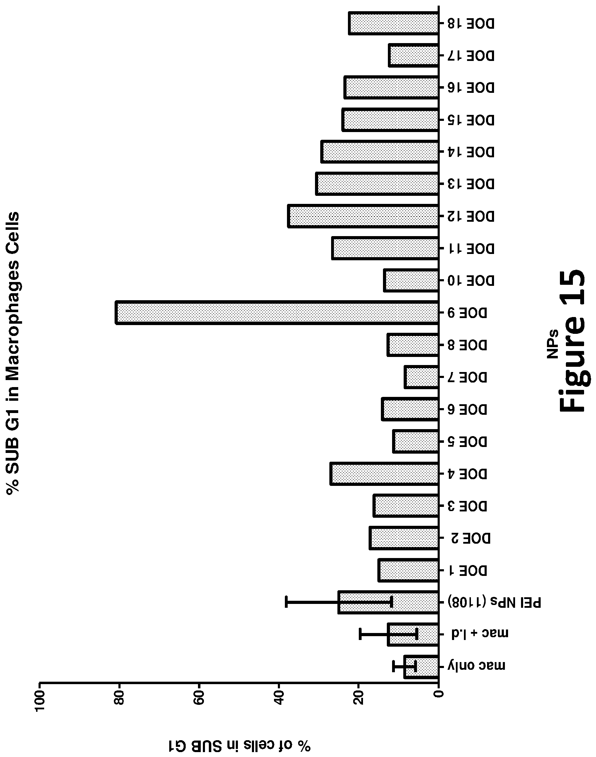

All 18 NPs were tested for their toxicity toward macrophages as measured by DNA fragmentation as assessed by FACS (**Fig. 15**). Many of the compositions were not at all detrimental to macrophage survival thus showing a superiority to the previous composition. Next, the NPs were assayed for their ability to kill promastigots and amastigots and MTT assay was performed to confirm Macrophage survival. The assays were performed as follows:

**Promastigote assay:** Promastigotes of *L.donovani,* one million in one ml of culture medium was incubated with 0.5µg of nanoparticle and incubated at 27Cc for 24 hours. The live cells were counted after 24 hours and the absolute percentage as compared to untreated control is represented in percentage.

**Amastigote assay:** Macrophage were transformed from THP1 monocyte cells cultured in RPMI medium by addition of 50ng/ml of Phorbol 12-Myristate 13- Acetate and incubated for 48 hours. After transformation into macrophages, the cells were infected with 5 million GFP tagged- *L.donovani.*

After incubation for 24 hours, the culture medium was washed and fresh medium was added along with 0.5µg of nanoparticles. The treated and untreated macrophages were observed under 20x microscope under live conditions after 24 hours. The GFP positive amastigotes inside macrophages as seen in the representative image of amastigote assay, were counted and converted to percentage in respect to the infected untreated cells.

**MTT assay:** THP1 monocytes were incubated with 0.5µg c of NP and incubated for 24hours. The cells were centrifuged at 1200rpm for 10minutes and suspended in 100 µL of fresh medium containing the MTT reagent (Sigma-Aldrich) (0.5 mg mL⁻¹) was added to each well followed by 20 min incubation. After incubation, MTT was removed, and 50 µL of DMSO (Sigma-Aldrich) was added to each well. All the absorption values were measured at 570 nm (Synergy 4, Biotek) and normalized to control. The graph represents mean of three different biological repeats and the statistical significant.

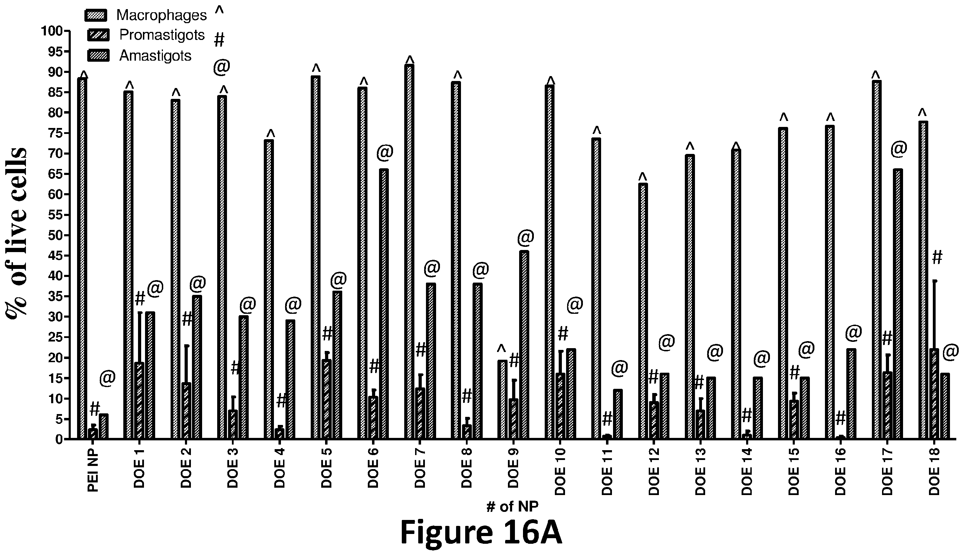

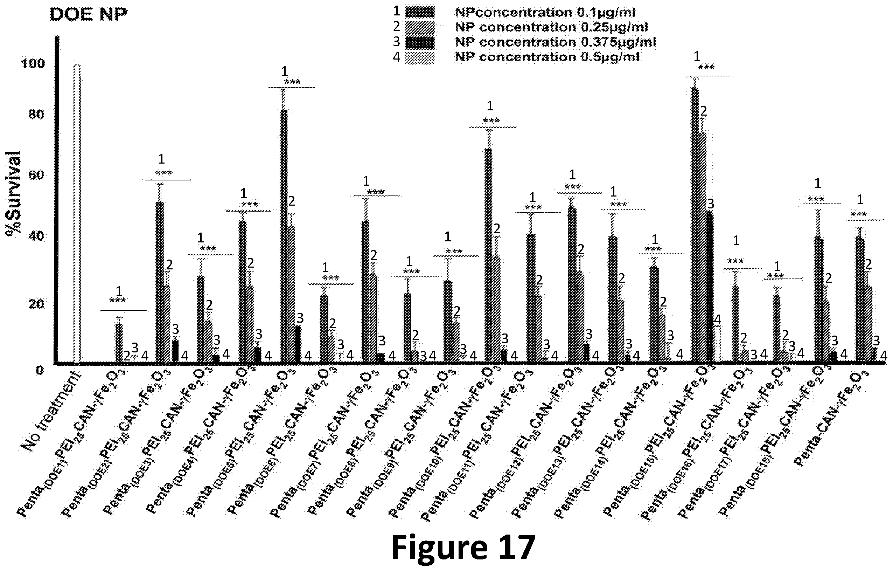

The results are presented in **Figures 16A** and summarized in **Table 4.** The promastigote assay was also preformed in triplicate with varying concentration of the NPs (**Fig. 17**).

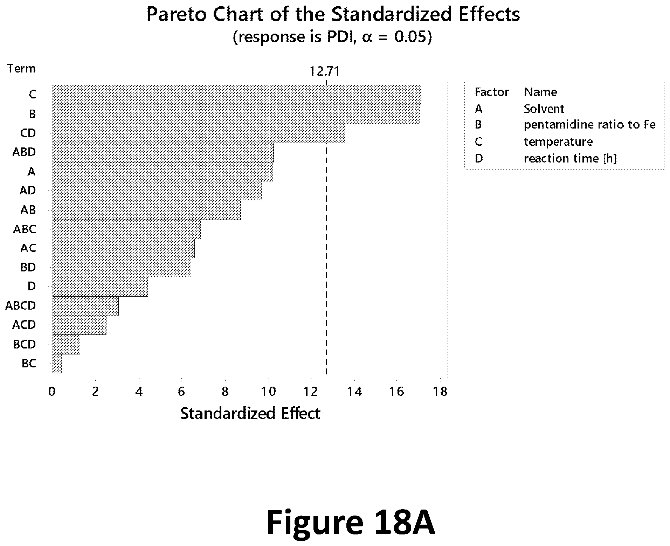

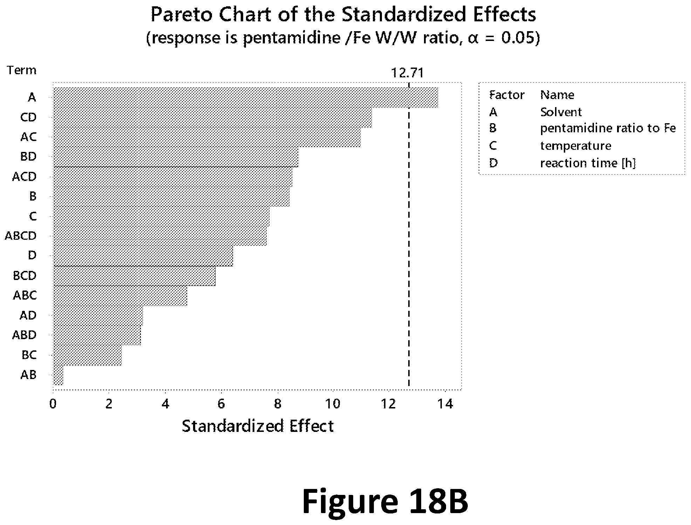

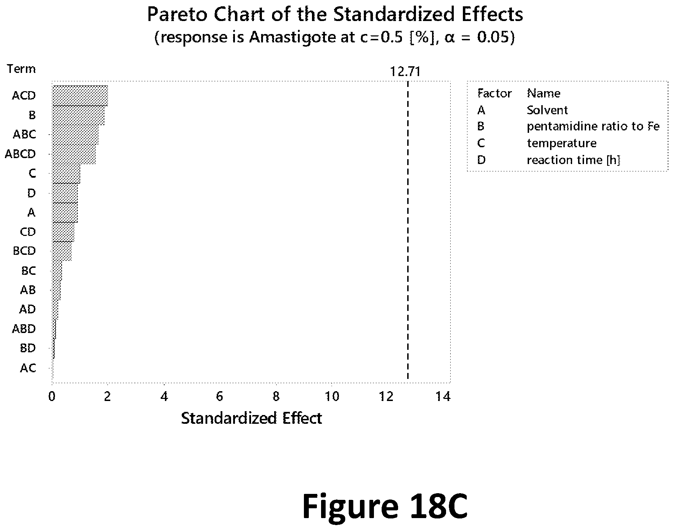

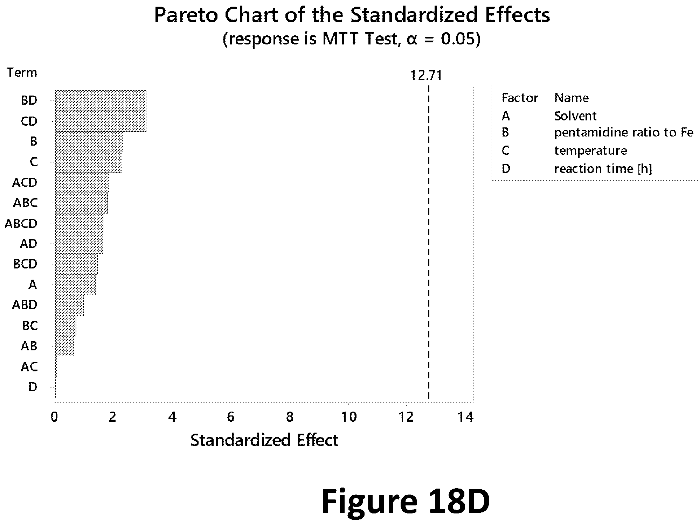

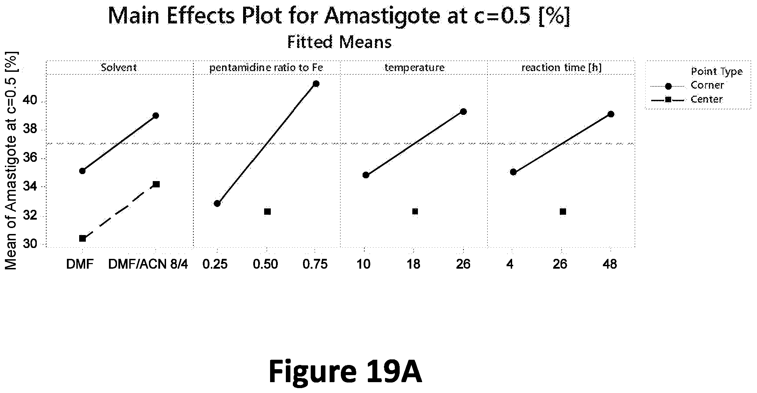

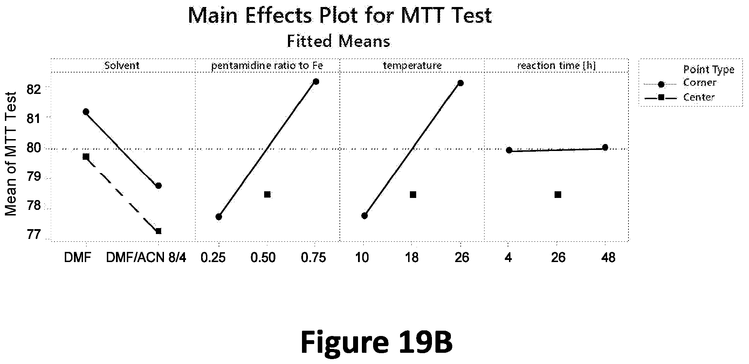

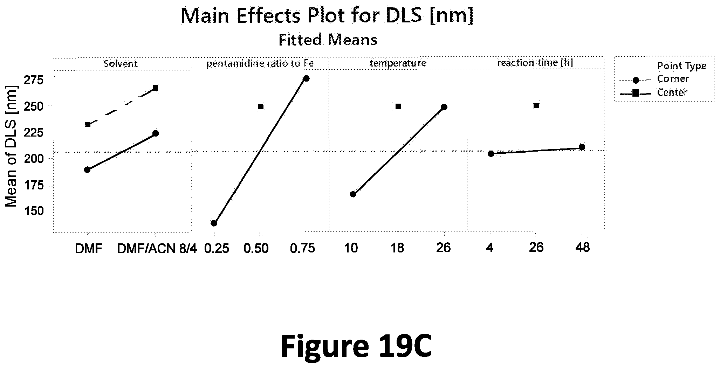

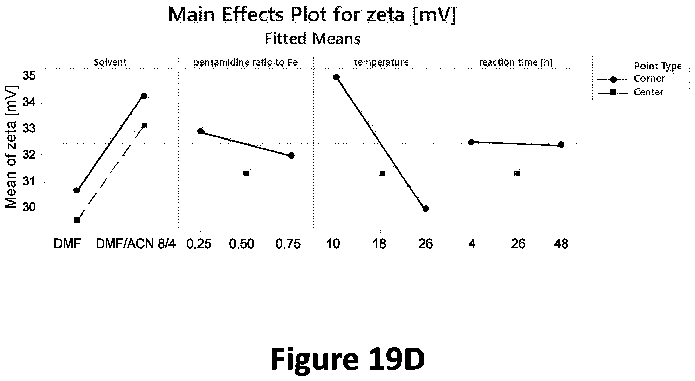

Following this, an extensive study done using the Minitab software. First Pareto chart was checked for all the responses (four of them are presented in figure13). In Pareto chart, any response or interaction that crosses the red line is statistically significant. In the case of Amastigotes and MTT assays (**Fig. 18C****,** **18D**), none of the factors or interaction was found statistically significant. Different results were observed for the PDI and for the final pentamidine/Fe ratio responses (**Fig. 18A****,** **18B**). Amongst all four investigated DoE factors (DoE-software analysis), the most influential one considering PDI was the *temperature,* then the *pentamidine*/*Fe used ratio,* and finally an effective interaction between the two factors, *i.e.,* the *temperature* and the *reaction time.* While for the *final pentamidine*/*Fe ratio* (**Fig. 18B**) only a single factor (solvent nature) is significant. Meaning, solubility issues are even more critical for the coordinative attachment of the pentamidine, then meaning the effective amount of pentamidine used in the reaction. In **Figures 19A-19D****,** main effect charts for four types of responses reveal the influence of each of these single factors, *via* corresponding graph slopes. In **Amastigotes** assays, the most influential single factor is the *pentamidine*/*Fe ratio* (as it possesses the highest slope), while the other three factors have been also found influential (**Fig. 19A**). In the case of the **MTT toxicity** assays, both *pentamidine*/*Fe ratio* and *temperature* factors are significant, however the *reaction time* one has a minor effect (**Fig. 19B**). Regarding both DLS (**Fig. 19C**) and ζ potential (**Fig. 19D**) data, the most influential factors amongst all the DoE-analyzed ones have been found to be the *pentamidine*/*Fe ratio* and the *temperature* respectively.

**Table 4: DMF: dimethylformamide, ACN: acetonitrile**

| ***Sample*** | **Solvent** | **Pentamidine/F ratio** | **T (°C)** | **Reaction tim (hr)** | **Diameter (nm)** | **Zeta potential (mV)** | **PDI** | **Toxicity: host macrophage viability** | **Amastigote viability (%)** | **Promastigote viability [%]** |
|---|---|---|---|---|---|---|---|---|---|---|
| **1** | DMF | 0.75 | 10 | 4 | 129.7 | 56.2 | 0.427 | 85.1 | 31 | 18.67 |
| **2** | DMF/ ACN 8/4 | 0.25 | 26 | 48 | 183.4 | 61.3 | 0.433 | 82.9 | 35 | 13.67 |
| **3** | DMF | 0.25 | 10 | 4 | 131.8 | 66.6 | 0.535 | 83.9 | 30 | 7.00 |
| **4** | DMF | 0.75 | 10 | 48 | 157.5 | 50.8 | 0.339 | 73.1 | 29 | 2.33 |
| **5** | DMF/ ACN 8/4 | 0.75 | 26 | 48 | 146.2 | 55.8 | 0.344 | 88.8 | 36 | 19.33 |
| **6** | DMF | 0.25 | 10 | 48 | 240.8 | 35.7 | 0.445 | 86 | 66 | 10.33 |
| **7** | DMF/ ACN 8/4 | 0.75 | 10 | 4 | 130.6 | 45.5 | 0.42 | 91.61 | 38 | 12.33 |
| **8** | DMF | 0.25 | 26 | 48 | 100.9 | 41.4 | 0.481 | 87.4 | 38 | 3.33 |
| **9** | DMF | 0.5 | 18 | 26 | 145.4 | 45.1 | 0.316 | 19.2 | 46 | 9.67 |
| **10** | DMF/ ACN 8/4 | 0.75 | 26 | 4 | 263.6 | 36.2 | 0.397 | 86.5 | 22 | 16.00 |
| **11** | DMF | 0.25 | 26 | 4 | 180.9 | 40.5 | 0.367 | 73.5 | 12 | 0.67 |
| **12** | DMF/ ACN 8/4 | 0.5 | 18 | 26 | 267.3 | 39.7 | 0.365 | 62.4 | 16 | 9.00 |
| **13** | DMF/ ACN 8/4 | 0.25 | 10 | 4 | 150.4 | 46.7 | 0.374 | 69.5 | 15 | 7.00 |
| **14** | DMF | 0.75 | 26 | 4 | 107.4 | 51.8 | 0.395 | 70.8 | 15 | 1.00 |
| **15** | DMF | 0.75 | 26 | 48 | 117.8 | 51 | 0.41 | 76.1 | 15 | 9.33 |
| **16** | DMF ACN 8 | 0.25 | 26 | 4 | 106.2 | 41 | 0.45 | 76.6 | 22 | 0.33 |
| **17** | DMF ACN 8 | 0.25 | 10 | 48 | 732.2 | 56.7 | 0.67 | 87.7 | 66 | 16.33 |
| **18** | DMF ACN 8 | 0.75 | 10 | 48 | 125.8 | 53.6 | 0.38 | 77.7 | 16 | 22.00 |

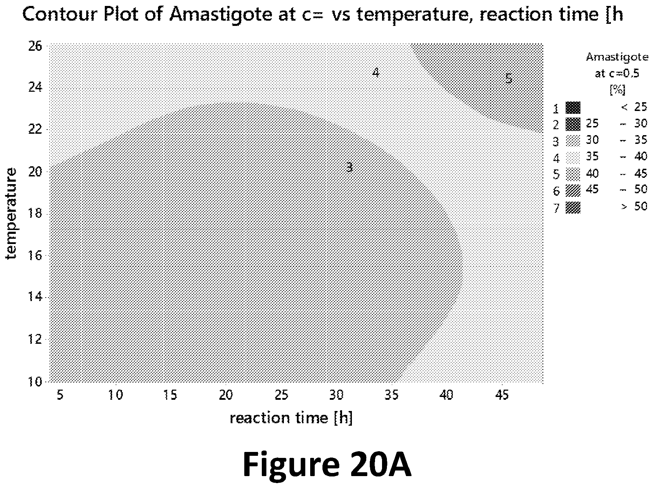

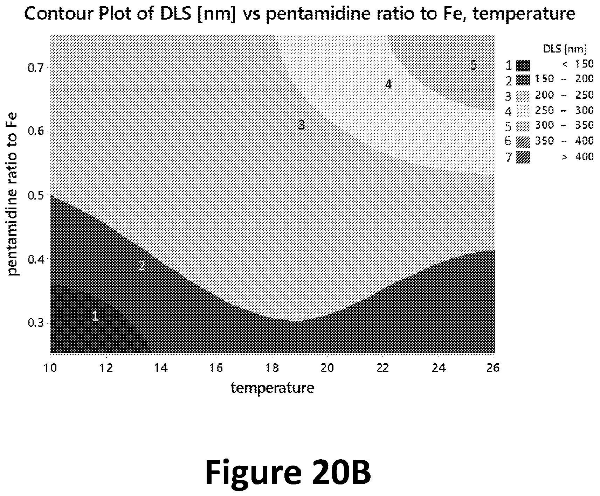

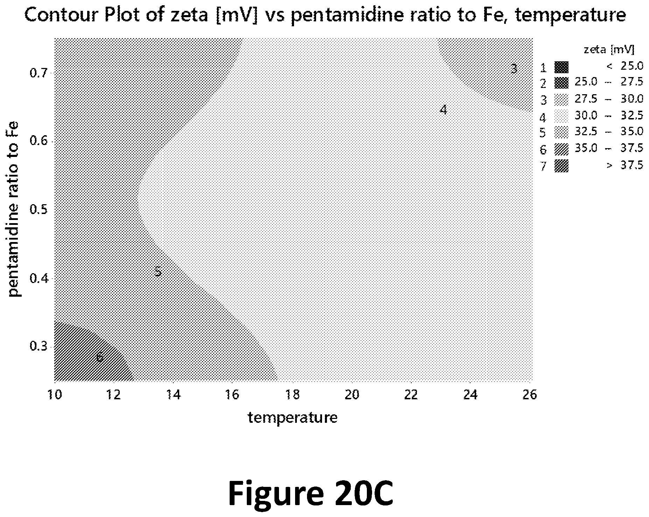

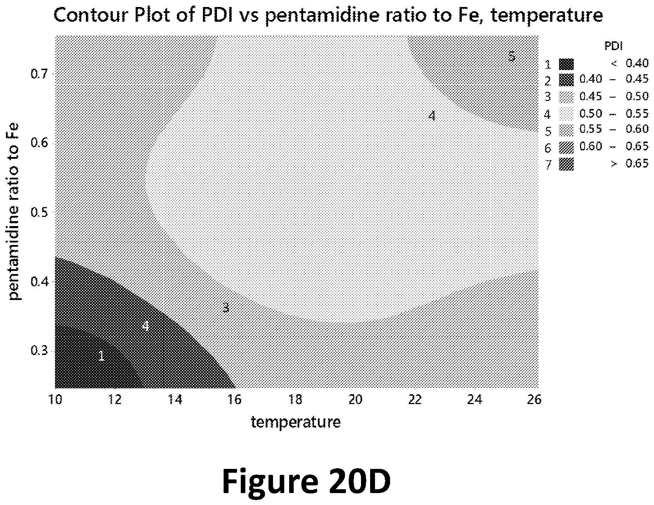

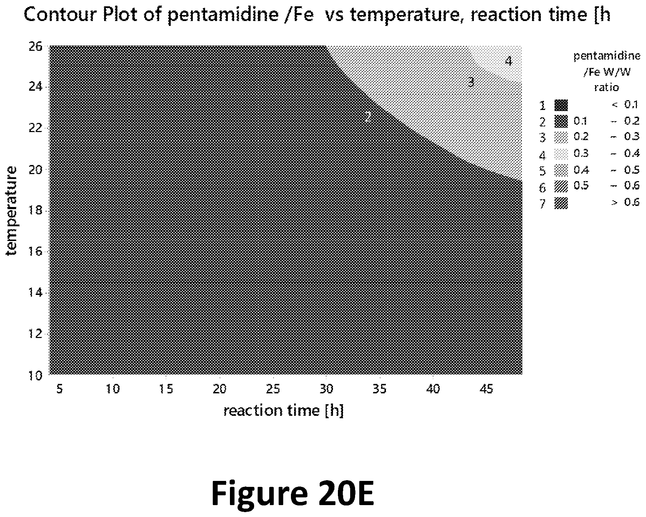

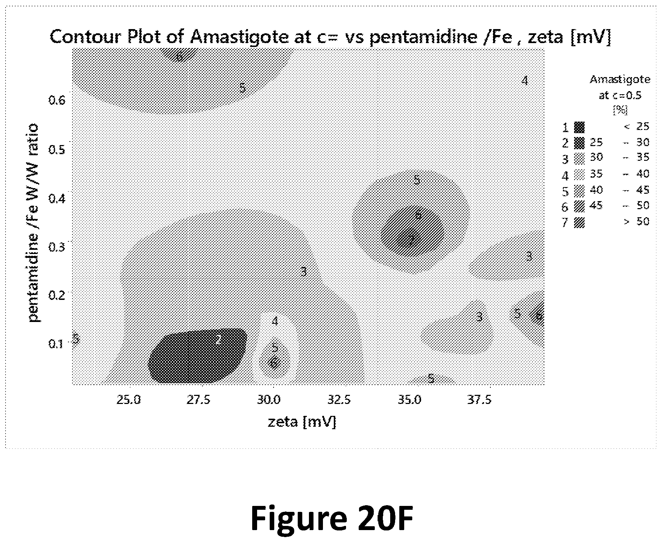

Moreover, **Figures 20A-20F** show the contour plots for the different responses. The 2D contour plots disclosing graphs similar to topographic maps showed two amongst reaction factors using both axes X & Y, while the chosen response is color-encoded at Z axis. From examination of these graphs, several interesting conclusions might be drawn. For the amastigote biology assays, according to the contour plots on **Figure 20A****,** the optimal reaction conditions are at a temperature lower than 20 *°C and* under 35 h. Based on the counter plots we can see the influence of the temperature and the amount of pentamidine on the chemical characterization; the DLS size, the ζ potential and the final pentamidine/Fe w/w ratio (**Fig. 20B-20D**, respectivly). For example, high amount of pentamidine in the reaction results in increasing the DLS size; and decreasing its ζ potential and PDI. In order to bind high amount of pentamidine to the CAN-γ-Fe2O3 NPs, we thus need to increase the temperature and the reaction time (This is accordance with **Fig. 20E**), however **Figure 20F** (which presents the effect of two chemical responses - Final pentamidine/Fe w/w ratio and ζ potential on the amastigote biology assay) illustrate that a high amount of pentamidine unexpectedly does not produce the best NPs against amastigotes. In total this data show that the most effective NPs will be one with ζ potential of about 27 mV and lower than 0.1 pentamidine/Fe w/w final ratio.

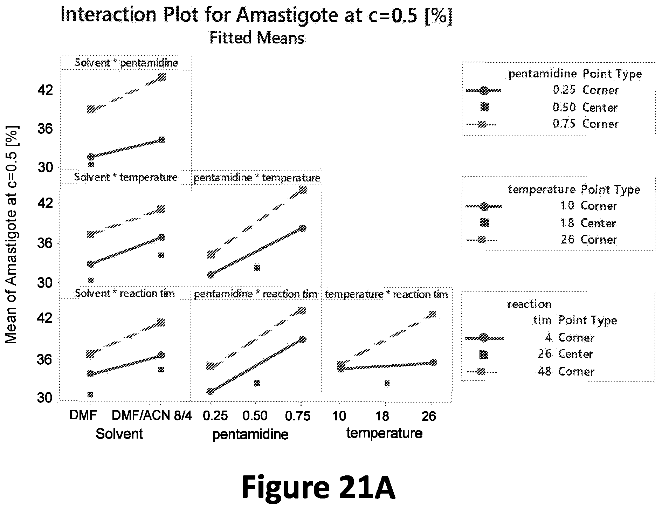

**Figures 21A-21B** presents the integration plots for amastigotes and MTT biological assays respectivly. An interaction plot basically reveals if there is an effective interaction between two different factors/reaction conditions for a certain given response. When the two considered graphs are either super-imposed or parallel, it means that there is no interaction between these two factors. On the contrary, line intersection indicates a strong interaction between these two considered factors.

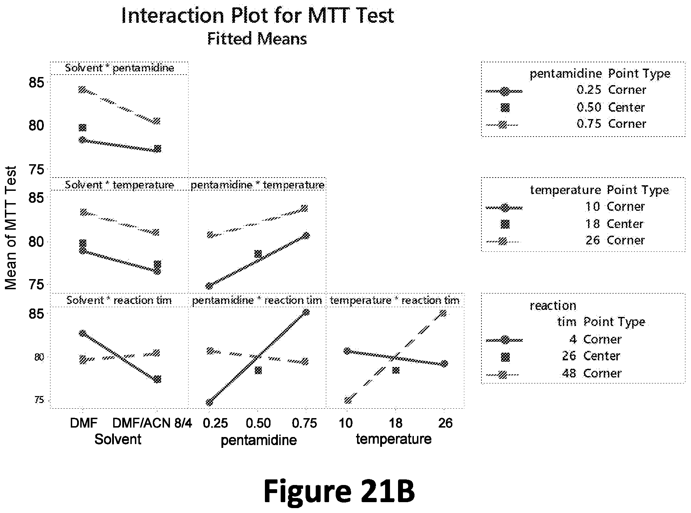

The interaction plots on **Figure 21A** disclosed that none of these factors have strong interactions on the amastigotes, while on the MTT toxicity results (**Fig. 21B**), some interactions are visible such as between pentamidine/Fe ratio and reaction time factors and between temperature and reaction time.

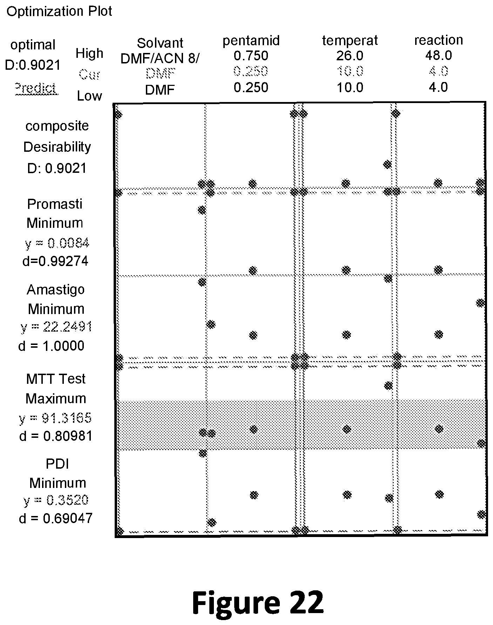

In order to calculate an optimal set of reaction conditions, the MINITAB software optimizer tool was used, and the corresponding importance of DoE-responses defined. The most important responses were set to be the biological assays for amastigote cells (set to be less than 30%) as well as the MTT toxicity assay (set to be above 80%). The biological promastigote assays and PDI were also optimized but with less importance. The outputs of the optimizer use/exploitation have been described in **Figure 22****.** The optimal solvent system was determined to be the DMF, while the pentamidine ratio to Fe was set at 0.75, with a 4 h reaction at 10°*C*. These reaction conditions are corresponding to the conditions set n°1 of the DoE-experimental array.

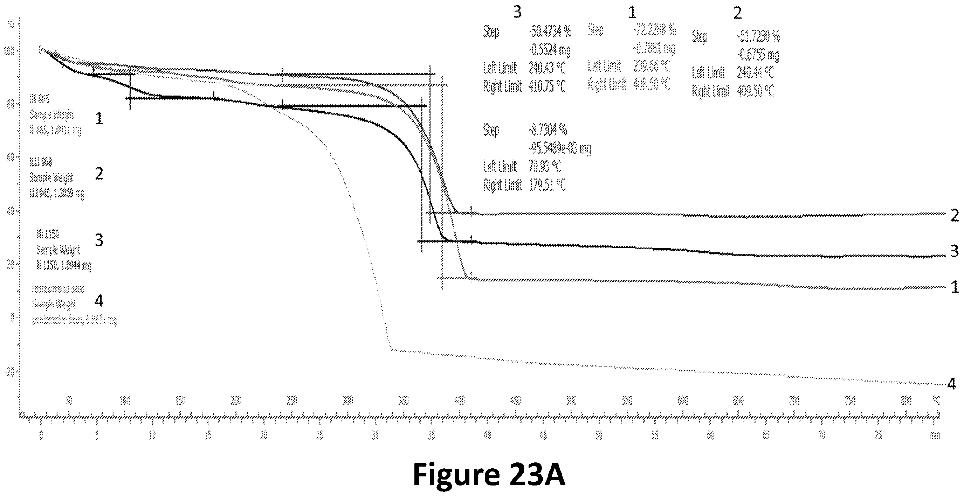

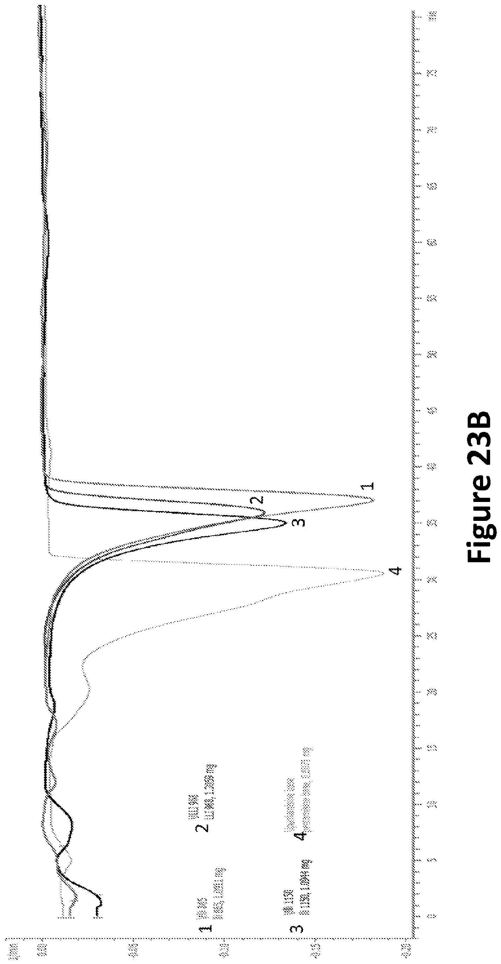

Such specific DoE 1 NPs were fabricated again using experimental conditions of the predicted DOE-optimized (similar to DOE 1 set of conditions), and the following NPs characteristics were obtained: a hydrodynamic NPs diameter of 165 nm with a polydispersity index of 0.35 and a ζ potential of +42.4 mV. Thermogravimetric TGA analyses have been also performed (**Fig. 23A**) measuring the PEI phase amounts (240-410 ⁰C temperature range) of DoE optimized *_{con}*PEI₂₅-Penta-CAN-*γ*-Fe₂O₃ NPs (50.47%), *_{con}*PEI₂₅-CAN-*γ-*Fe₂O₃ NPs (73.23%), and DoE optimized *_{con}*PEI₂₅-CAN-*γ*-Fe₂O₃ NPs (51.72%). Interestingly, there only 1.25% difference in PEI quantities between *_{con}*PEI₂₅-Penta-CAN-*γ-*Fe₂O₃ and DoE optimized *_{con}*PEI₂₅- CAN-*γ*-Fe₂O₃ NPs. However, there is a significant difference in the biological results including the NPs toxicity between these two types of NPs. An additional weight drop of 8.73% can also be seen for the DoE optimized *_{con}*PEI₂₅-Penta-CAN-γ-Fe₂O₃ NPs in the temperature range of 70-180 ⁰C that is not shown for the sole *_{con}*PEI₂₅-CAN-*γ*-Fe₂O₃ NPs (**Fig. 23B**, 1^{st} derivative).

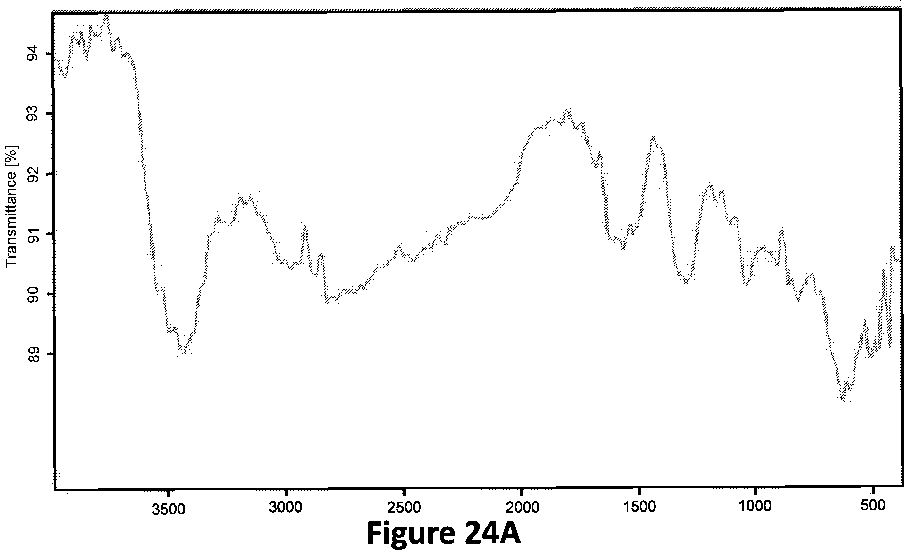

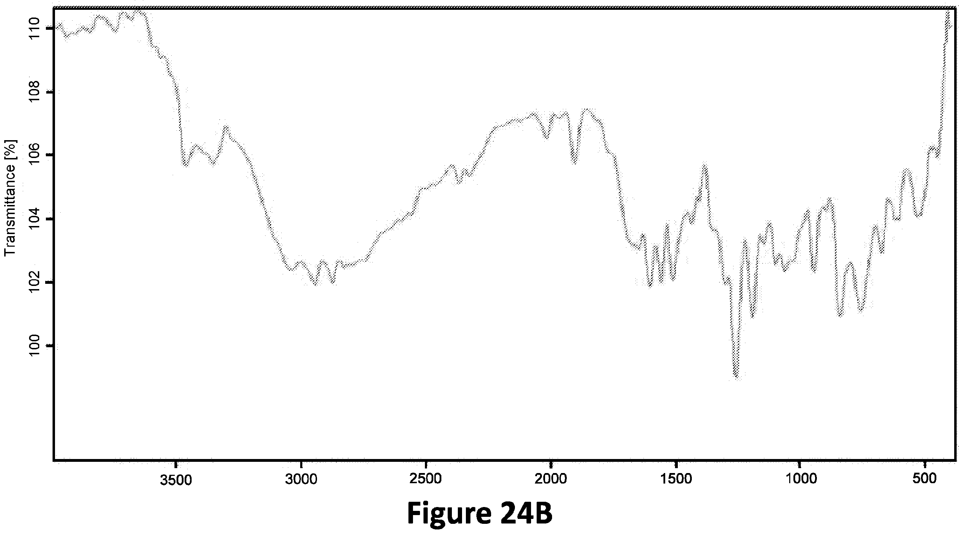

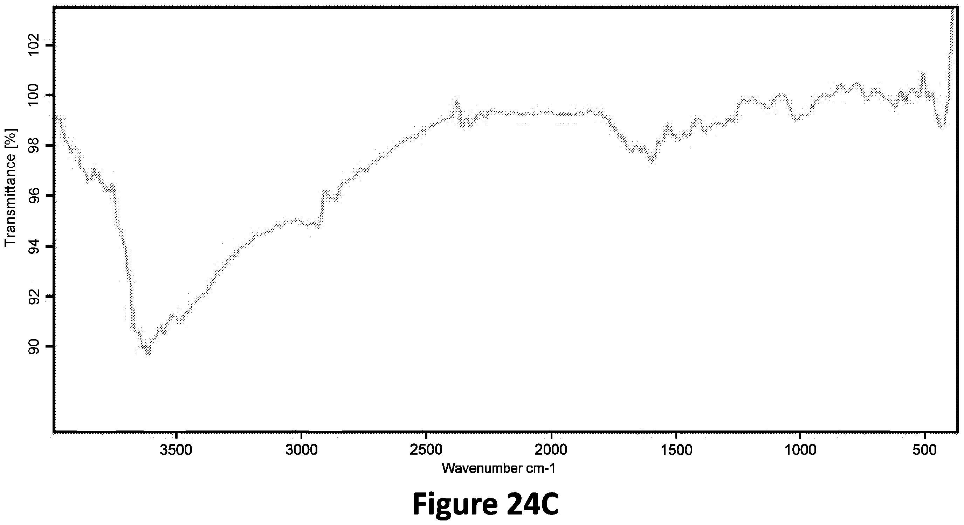

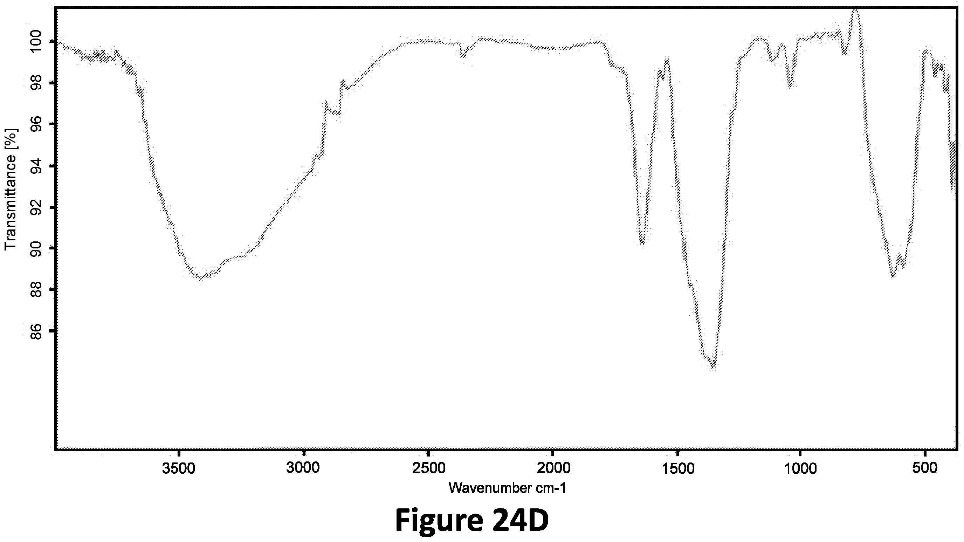

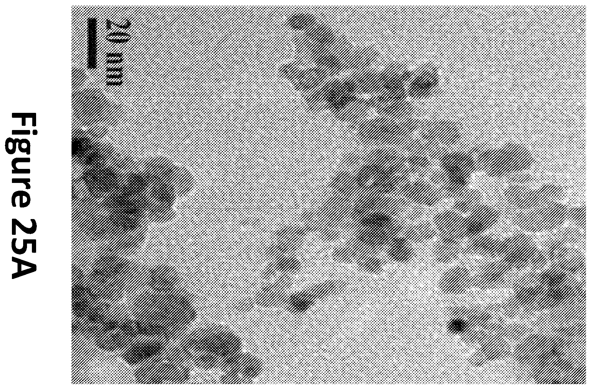

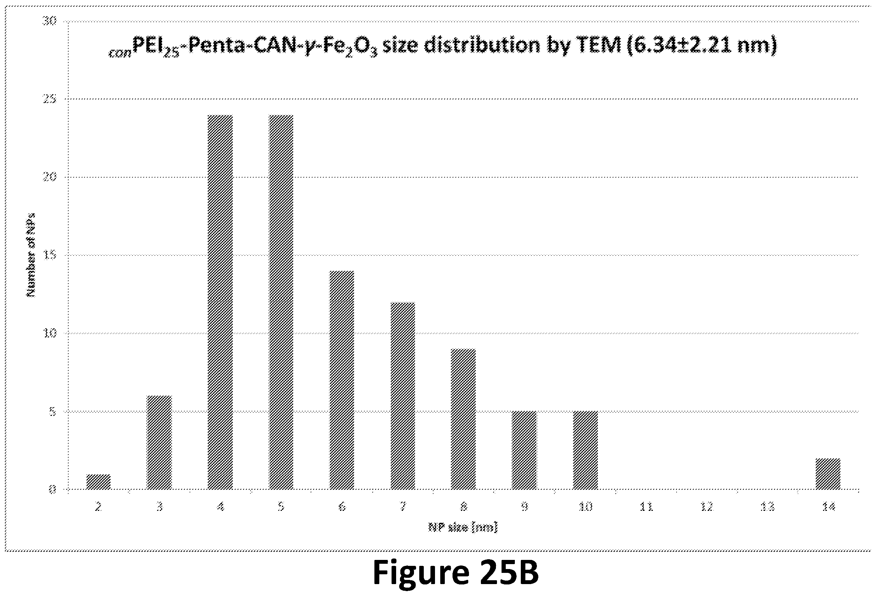

Characterization FTIR spectra were also collected for DoE-optimized *_{co n}*PEI₂₅-CAN-*γ*-Fe₂O₃ NPs, CAN-maghemite NPs, and also for simple *b*-PEI₂₅ and pentamidine basic compounds/phases (**Fig. 24A-24D**, respectively). Distinctive peaks of CAN-maghemite *b*-PEI₂₅ and pentamidine base can be found in the *_{con}*PEI₂₅- CAN-*γ*-Fe₂O₃ FT-IR spectrum which confirms the presence of both pentamidine and b-PEI₂₅ on the CAN-maghemite surface. For example, FT-IR absorption peaks at 3551, 3494 and 1673 cm⁻¹ can be selectively attributed to *b*-PEI₂₅, while peaks at 2941, 1602, 1559, 1517 and 854 cm⁻¹ might be fully characterizing the pentamidine drug together with peaks at 1355 and 623 cm⁻¹ that are typical CAN-maghemite peaks. In **Figure 25A**, TEM image as well as size distribution analysis by TEM (**Fig. 25B**) reveals obtainment of a 6.34±2.21 nm round nanoparticle.

In addition, to quantify how much pentamidine was actually coordinatively attached onto the NPs surface within the PEI phase, HPLC analysis has been carried out as described before. The pentamidine/Fe W/W ratio was calculated to be 0.12, very similar to the original DOE 1- hybrid NPs.

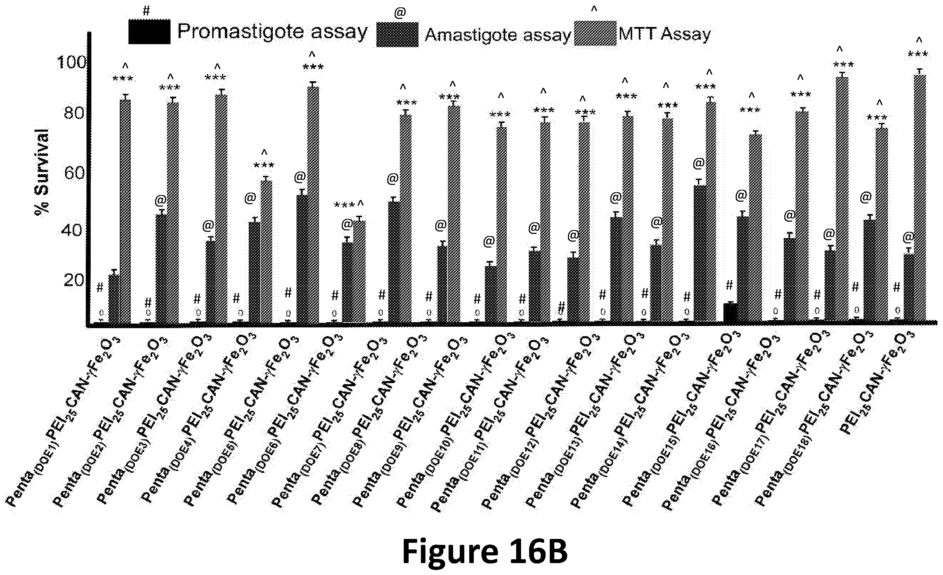

The new batch of optimized NPs was tested again in all biological assays (see **Fig. 16B**). The MTT toxicity assay for macrophages cells treated with 0.5 µg/ml of the optimized NPs showed that 83% of cells were alive. The same amount of DoE-optimized NPs was incubated with macrophages infected with parasites (amastigotes) or promastigotes and the number of parasites was then counted. Such results showed almost 100% killing of promastigotes and 76% killing of amastigotes.

***In vivo* studies:** Acute toxicity was assessed by mortality of mice within 24 hours following NPs injection or by the evaluation of diverse hematological and biochemical parameters (effect on the chemistry of the blood, hematological changes, as well as changes in liver enzymes) after 7 and 28 days. For the evaluation of hematological and biochemical parameters, blood samples were collected. 0.4 mg of optimized NPs (sample 10 in **Table 2** or DOE10) was injected intravenously for per kg (based on the amount of iron).

All animal experiments were performed in compliance with the Guidelines for the Care and Use of Research Animals established by the Bar-Ilan University Animal Studies Committee. Head of the Bar-Ilan University Study Approval no. 06-01-2015. BALB/c mice (Harlan Laboratories Israel Ltd., Jerusalem, Israel). Mice aged 8-9 weeks were intravenously injected in the tail vein with nanoparticle of concentration 0.4mg/kg bodyweight as indicated above. Each group was composed of 8 mices. For the evaluation of hematological parameters, 150 µl of blood were collected in EDTA-coated tubes. For the evaluation of biochemical parameters, 350 µl of blood were collected in non-coated gel tubes, centrifuged (4000g, 4 min, RT) and serum separated. Then, EDTA-coated tubes and serum samples were transferred to American Medical Laboratories (AML, Herzliya Medical Center, Israel) for further analyses.

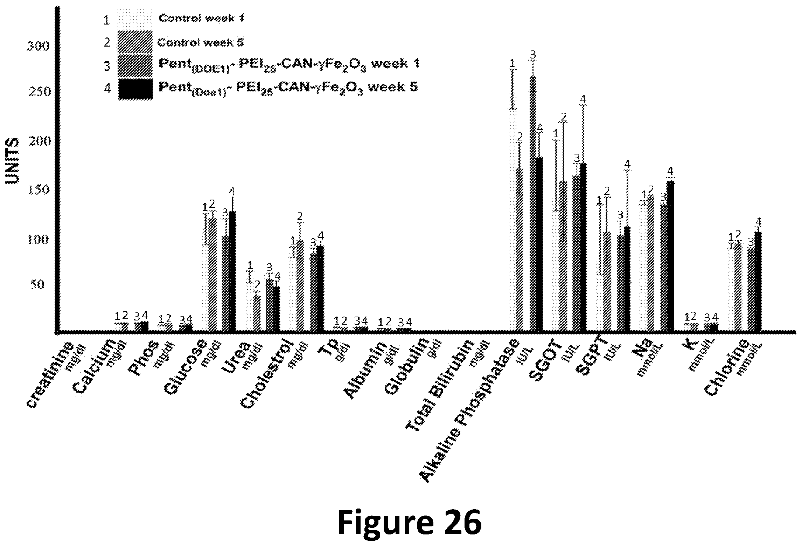

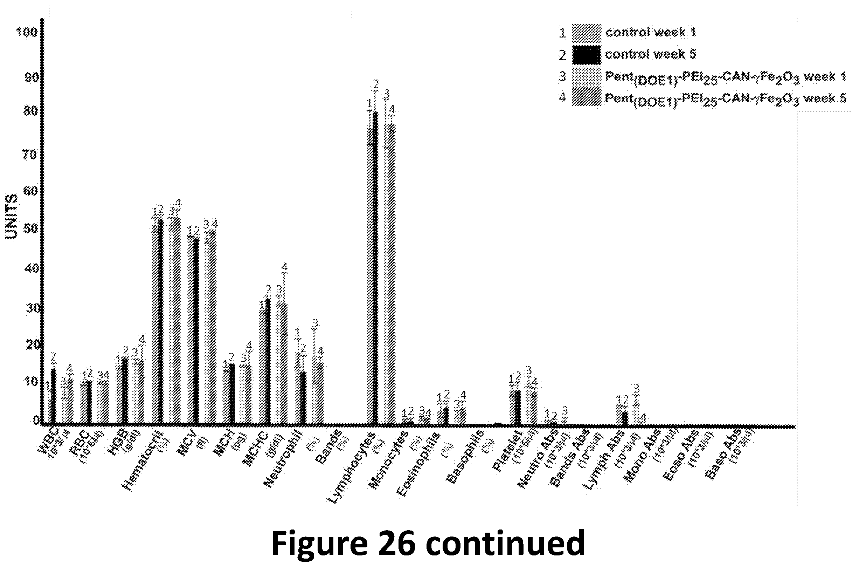

It was concluded that hematology, chemistry and blood biochemistry showed no significant difference in parameters between the control and the mice-injected with the different NPs (**Fig. 26****, Table 5**).

**Table 5**

| **Biochemistry** | **Water** | | **DOE10** | | **Water** | | **DOE10** | | **p-value** |
|---|---|---|---|---|---|---|---|---|---|
| | **Average Week 1** | **STD** | **Average Week 1** | **STD** | **Average Week 4** | **STD** | **Average Week 4** | **STD** | |
| **Total Protein (g/dl)** | 5.48 | 0.21 | 5.62 | 0.36 | 5.23 | 0.20 | 4.54 | 1.26 | 0.53129 |
| **Albumin (g/dl)** | 4.05 | 0.18 | 4.10 | 0.33 | 3.63 | 0.15 | 3.10 | 1.03 | 0.53129 |
| **Globulin (g/dl)** | 1.43 | 0.24 | 1.52 | 0.09 | 1.61 | 0.13 | 1.44 | 0.23 | 0.53129 |
| **Total Bilirubin (mg/dl)** | 0.14 | 0.19 | 0.06 | 0.02 | 0.31 | 0.42 | 0.10 | 0.07 | 0.75400 |
| **ALP=Alkaline Phosphatase (IU/L)** | 227.00 | 18.14 | 197.33 | 12.88 | 180.50 | 13.18 | 175.50 | 32.89 | 0.53129 |
| **AST=SGOT (IU/L)** | 111.00 | 30.22 | 150.67 | 57.55 | 145.25 | 49.89 | 205.00 | 170.05 | 0.85621 |

Furthermore, stationary phase promastigote (10⁸) of *L. donovani* in 200µl PBS were injected to the tail vein of BALB /c mice on day 0 and sacrificed on day 21. The liver was dissected aseptically and cultured in M199 and RPMI media. The passaged parasites obtained were re-injected through mouse tail vein. The 10^{th} passage (p10) was used in the experimental infection. Three weeks post-infection, the mice were sacrificed and the liver dissected. One fourth of liver was dissected under sterile conditions and DNA preparation was done using standard conditions.

The scheme of the in vivo experiment was as follows: Female BALB/c mice were blindly grouped into 5 cohorts (n=8); Uninfected control; Infected-untreated; Infected-Treated with Pent_{(DOE1)}-PEI₍₂₅₎-CAN-γFe₂O₃; Infected-Treated with PEI₍₂₅₎-CAN-γFe₂O₃; Infected-Treated with PEI_{(25OX)}-CAN-γFe₂O₃. The PEI_{(25OX)}-CAN-γFe₂O₃ NPs were described by us previously (Lellouche et al., Bioconjug. Chem. 26: 1692-701, 2015). These NPs carry the PEI that was oxidized by H₂O₂ to reduce potential toxicity. All the cohorts except the uninfected control were injected with stationary phase promastigotes of passage10 (p10) *L.donovani* by intravenously through the tail vein (1× 10⁷ parasites per mouse in 100µl PBS). On day10, day14, the treated groups received intravenous injection to their tail vein. NPs were in sterile distilled water (200µl) at a concentration of 0.4mg/kg of mice body weight. On day 21 post infection, mice were sacrificed. Liver was dissected under sterile conditions and weighed.

The amount of parasite DNA was determined using real time qPCR of the Leishmania GP63 gene and using mouse BDNF gene as reference gene. The primers used were: (BDNF forward primer 5' CTGGATGCCGCAAACATGTC (SEQ ID NO: 1); BDNF reverse primer 5' CTGCCGCTGTGACCCACTC (SEQ ID NO: 2) and GP63 Forward 5' AGTACGGCTGCGACACCTTGGAG (SEQ ID NO: 3); GP63 Reverse primer 5' GTTCCGGCCCCACGGCATCACC (SEQ ID NO: 4)). 20 µl reaction mixture containing 100 ng DNA, 1× SYBR, 0.4 µM each primer was run in a CFX96 real time PCR machine was used. All samples were run in triplicate separately for the 2 primer sets and the PCR cycle including 30 s incubation step at 95 °C, then 40 cycles of 5 s at 95 °C and 30 s at 60 °C. The average Ct values (threshold cycle) and the standard deviation (SD) was determined by the BIORAD software of the instrument. Infection was also assessed microscopically using Giemsa-stained liver imprints by blinded counting of amastigotes and calculation of Leishman-Donovan Units (LDU) by the formula (amastigotes per 500 host nuclei × weight of liver in grams).

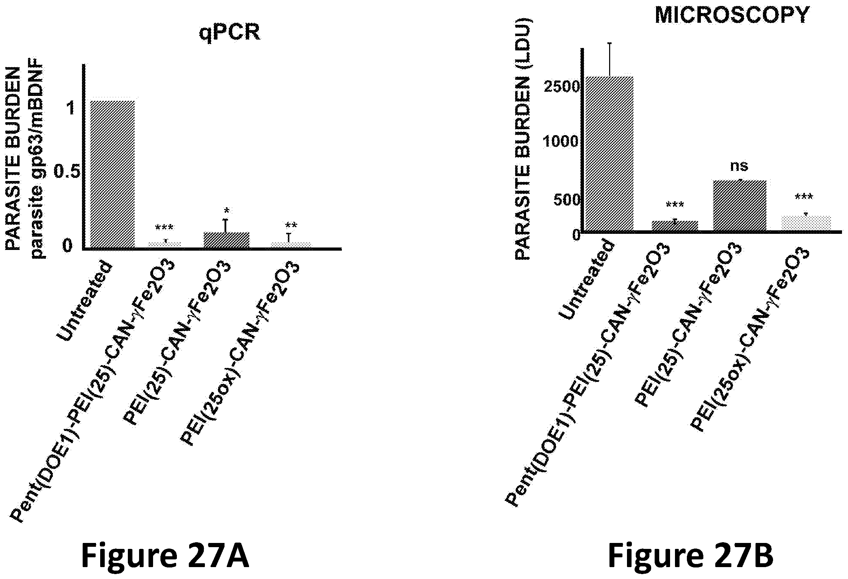

The relative quantification of parasite GP63 versus mouse BDNF by qPCR is depicted in **Figure 27A** and the values were generated by BIORAD qPCR software. The parasite burden as estimated by quantification of GP63 over mBDNF (Relative normal expression) were 1.0 +/- 0.131 for the untreated, 0.024 +/- 0.00248 for Pent_{(DOE1)}-PEI₍₂₅₎-CAN-γFe₂O₃, 0.113 +/- 0.0873 for PEI₍₂₅₎-CAN-γFe₂O₃ and 0.063 +/- 0.0084 for PEI₍₂₅ₒₓ₎-CAN-γFe₂O₃ NPs. One way ANOVA yielded a p-value of 0.0000851 and followed by Tukey post hoc test. The p-values were 0.0000492 for Pent_{(DOE1)}-PEI₍₂₅₎-CAN-γFe₂O₃; 0.011560 for PEI₍₂₅₎-CAN-γFe₂O₃; & 0.0023597 for PEI₍₂₅ₒₓ₎-CAN-γFe₂O₃ NPs. The mouse reference gene (mBDNF) of both treated and untreated did not show significance difference indicating treatment did not affect it.

The microscopy analysis is summarized in **Figure 27B****.** The Leishman-Donovan units representing the parasite burdens were 2473+/- 678 for untreated, 178+/- 35 for Pent_{(DOE1)}-PEI₍₂₅₎-CAN-γFe₂O₃ NPs treatment, 766+/- 102 for PEI₍₂₅₎-CAN-γFe₂O₃ NPs and 227+/- 81 for PEI₍₂₅ₒₓ₎-CAN-γFe₂O₃ ones. In Giemsa, one way ANOVA of the data yielded p value (0.0000925) indicating significance due to treatment and the p-values by post hoc Tukey test were 0.0004604 for Pent_{(DOE1)}-PEI₍₂₅₎-CAN-γFe₂O₃; 0.55 for PEI₍₂₅₎-CAN-γFe₂O₃; 0.0006264 for PEI₍₂₅ₒₓ₎-CAN-γFe₂O₃ NPs. Pearson correlation coefficient between Giemsa LDU and qPCR was 0.989951.

In summary, *Leishmania* infection was developed after 10-14 days. Using NPs at 0.4mg/kg of mouse body weight and upon three injections as described above, the functional NPs completely eliminated the parasites (based on qPCR), but Pent_{[DOE1]}-PEI₂₅-CAN-γ-Fe₂O₃ NPs have a slight advantage over PEI₂₅-CAN-γ-Fe₂O₃ or PEI₂₅ₒₓ-CAN-γ-Fe₂O₃ NP as assessed by the Giemsa assay.

To be able to examine the utilization of the nano-drug for cutaneous *Leishmaniasis,* we examined if placing the drug near a migrating *T. brucei* cells will affect motility. To assess the biological activity of the drug we prepared the drug both in liquid and ointment for the following NPs: Pent_{[DOE1]}-PEI₍₂₅₎-CAN-γ-Fe₂O₃, PEI₍₂₅₎-CAN-γ-Fe₂O₃, PEI₍₂₅ₒₓ₎-CAN-γ-Fe₂O₃, and CAN-γ-Fe₂O₃ NPs.

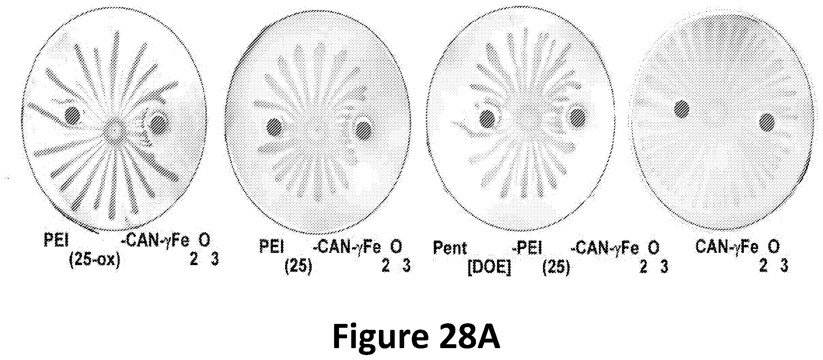

These four fabricated NPs were checked on plates with both Fe concentrations of 0.02 and 0.2 mg/ml. The results shown in **Figure 28A** demonstrate that the wild-type colony approaching the nanoparticle stopped moving in a concentration dependent manner.

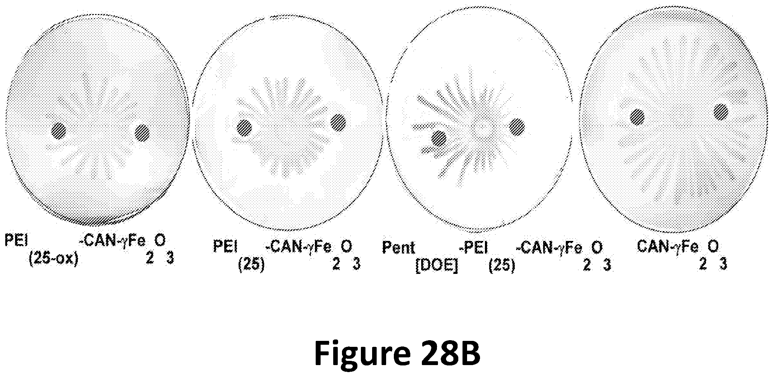

The nano-drugs ointments were prepared as following: 0.02% and 0.2% of Fe to a base ointment (weight percentages) for each of the four fabricated NPs were taken, and then diluted (if needed) to a final ratio of 2:1 water per cream. The NPs final solution was added to the cream and stirred till a homogeneous texture was obtained. The wild-type colony stopped while approaching 0.2% ointment and progressed in case of 0.02% showing concentration dependence (**Fig 28B**). After observing that the NPs in ointment are biological active, we tested their ability to prevent the development of the cutaneous disease in a mouse model.

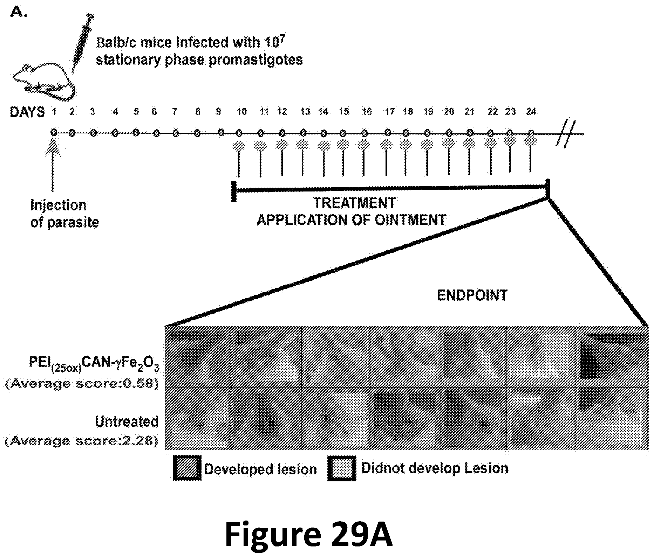

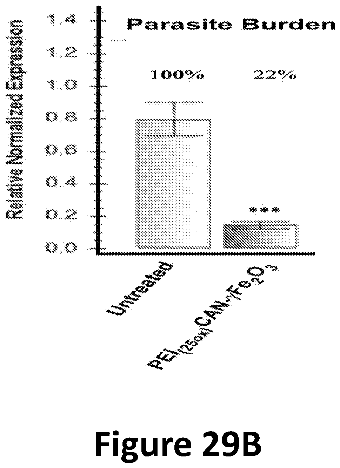

Female BALB/c mice were blindly grouped into 3 cohorts (n=7); Infected-untreated; Infected-Treated with PEI_{(25OX)}-CAN-γFe₂O₃ NPs. The cohorts were injected with stationary phase *L. major* promastigotes. Treatment with application of ointment with cotton applicator was started on day10 until day 24 during which the site of experimental infection of the animals were smeared with ointment (having 0.2% of the nanoparticles). The pictures of the mice were taken at the end of the experiment and parasites were taken from the lesions and compared to lesion formed in infected mice (**Fig. 29A**). The amount of Leishamanis DNA was determined by qPCR as descried in previously (**Fig. 29B**). In summary, the ointments with NPs given daily post-infection were able to reduce the disease by up to 80% as determined by qPCR.

Although the invention has been described in conjunction with specific embodiments thereof, it is evident that many alternatives, modifications and variations will be apparent to those skilled in the art.

## Claims

1. A composition comprising at least one particle, said at least one particle having a core comprising metal oxide, and a shell comprising an ion of a lanthanide element, wherein said lanthanide element is linked to a polymer and to the active agent pentamidine, and wherein a weight ratio of said active agent to said metal oxide is in the range of from 1:2 to 3:2, respectively.

2. The composition of claim 1, wherein said metal oxide is: iron oxide comprising a maghemite γ-Fe₂O₃ phase.

3. The composition of any one of claims 1 to 2, wherein said lanthanide element is selected from the group consisting of: cerium, lanthanum, praseodymium, neodymium, promethium, samarium, europium, gadolinium, terbium, dysprosium, holmium, erbium, thulium, ytterbium, lutetium, and any combination thereof.

4. The composition of any one of claims 1 to 3, wherein said polymer: (a) is selected from the group consisting of: polyethylenimine (PEI), poly-L-lysine, chitosan, poly-L-arginine, dendrimeric polyamidoamine (PAMAM), polyacrylate, polyphosphate, polyethyleneglycol (PEG), and any combination thereof; or (b) molecular weight is in the range of from 500 Da to 60 KDa.

5. The composition of any one of claims 1 to 4, wherein a hydrodynamic diameter of said particle is in the range of from 60 nm to 2 µm.

6. A pharmaceutical product comprising the composition of any one of claims 1 to 5, and a pharmaceutically acceptable carrier, excipient or adjuvant.

7. The pharmaceutical product according to claim 6, for use in treating an infectious disease or disorder.

8. The pharmaceutical product for use according to claim 7, wherein said disease or disorder is associated with parasitic infection.

9. The pharmaceutical product for use according to any one of claims 6 to 8, being in the form selected from the group consisting of: paste, cream, lotion, foam, gel, emulsion, ointment, and soap.

10. A method for obtaining a composition comprising at least one particle, having a core comprising metal oxide, and a shell comprising an ion of a lanthanide element, wherein said ion of said lanthanide element has linked thereto a polymer and pentamidine, the method comprising the steps of:
a. mixing a solvent, said lanthanide element, and a plurality of particles of said metal oxide, thereby obtaining a suspension, and
b. adding said polymer and said pentamidine to said suspension.

11. The method of claim 10, wherein the mixing step is performed under ultrasonic agitation.

12. The method of claim 10, wherein said adding said polymer and said pentamidine is performed simultaneously.

13. The pharmaceutical product for use according to claim 8, wherein said parasitic infection comprises Leishmania infection.

## Patentansprüche

1. Zusammensetzung, die mindestens ein Partikel umfasst, wobei das mindestens eine Partikel einen Kern aufweist, der ein Metalloxid umfasst, und eine Hülle, die ein Ion eines Lanthanidelements umfasst, wobei das Lanthanidelement mit einem Polymer und mit dem Wirkstoff Pentamidin verbunden ist, und wobei ein Gewichtsverhältnis des Wirkstoffs zum Metalloxid jeweils im Bereich von 1:2 bis 3:2 liegt.

2. Zusammensetzung nach Anspruch 1, wobei das Metalloxid ist: Eisenoxid, das eine Maghemit-γ-Fe₂O₃-Phase umfasst.

3. Zusammensetzung nach einem der Ansprüche 1 bis 2, wobei das Lanthanidelement aus der Gruppe bestehend aus Cerium, Lanthan, Praseodym, Neodym, Promethium, Samarium, Europium, Gadolinium, Terbium, Dysprosium, Holmium, Erbium, Thulium, Ytterbium, Lutetium und jeder beliebigen Kombination davon ausgewählt ist.

4. Zusammensetzung nach einem der Ansprüche 1 bis 3, wobei das Polymer: (a) aus der Gruppe bestehend aus Polyethylenimin (PEI), Poly-L-Lysin, Chitosan, Poly-L-Arginin, Polyamidoamin (PAMAM)-Dendrimer, Polyacrylat, Polyphosphat, Polyethylenglycol (PEG) und jeder beliebigen Kombination davon ausgewählt ist; oder (b) das Molekulargewicht im Bereich von 500 Da bis 60 KDa liegt.

5. Zusammensetzung nach einem der Ansprüche 1 bis 4, wobei ein hydrodynamischer Durchmesser des Partikels im Bereich von 60 nm bis 2 µm liegt.

6. Pharmazeutisches Produkt, umfassend die Zusammensetzung nach einem der Ansprüche 1 bis 5 und einen pharmazeutisch akzeptablen Träger, Trägerstoff oder Wirkverstärker.

7. Pharmazeutisches Produkt nach Anspruch 6, zur Verwendung bei der Behandlung einer infektiösen Krankheit oder Störung.

8. Pharmazeutisches Produkt zur Verwendung nach Anspruch 7, wobei die Krankheit oder Störung mit einer parasitären Infektion zusammenhängt.

9. Pharmazeutisches Produkt zur Verwendung nach einem der Ansprüche 6 bis 8, das in der Form ausgewählt aus der Gruppe bestehend aus Paste, Creme, Lotion, Schaum, Gel, Emulsion, Salbe und Seife vorliegt.

10. Verfahren zum Erhalten einer Zusammensetzung, die mindestens ein Partikel umfasst, das einen Kern aufweist, der ein Metalloxid umfasst, und eine Hülle, die ein Ion eines Lanthanidelements umfasst, wobei das Ion des Lanthanidelements mit einem Polymer und mit Pentamidin verbunden ist, wobei das Verfahren die folgenden Schritte umfasst:
a. Mischen eines Lösungsmittels, des Lanthanidelements und einer Mehrzahl von Partikeln des Metalloxids, wodurch eine Suspension erhalten wird, und
b. Hinzufügen des Polymers und des Pentamidins zu der Suspension.

11. Verfahren nach Anspruch 10, wobei der Schritt des Mischens unter Ultraschall-Agitation durchgeführt wird.

12. Verfahren nach Anspruch 10, wobei das Hinzufügen des Polymers und des Pentamidins gleichzeitig durchgeführt wird.

13. Pharmazeutisches Produkt zur Verwendung nach Anspruch 8, wobei die parasitäre Infektion eine Leishmaniase-Infektion umfasst.

## Revendications

1. Composition comprenant au moins une particule, ladite au moins une particule présentant un noyau comprenant un oxyde métallique, et une enveloppe comprenant un ion d'un élément de lanthanide, dans laquelle ledit élément de lanthanide est lié à un polymère et à l'agent actif pentamidine, et dans laquelle un rapport en poids dudit agent actif audit oxyde métallique est dans la plage de 1:2 à 3:2, respectivement.

2. Composition selon la revendication 1, dans laquelle ledit oxyde métallique est : un oxyde de fer comprenant une phase de γ-Fe₂O₃maghémite.

3. Composition selon l'une quelconque des revendications 1 à 2, dans laquelle ledit élément de lanthanide est choisi dans le groupe consistant en : le cérium, le lanthane, le praséodyme, le néodyme, le prométhium, le samarium, l'europium, le gadolinium, le terbium, le dysprosium, l'holmium, l'erbium, le thulium, l'ytterbium, le lutétium, et toute combinaison de ceux-ci.

4. Composition selon l'une quelconque des revendications 1 à 3, dans laquelle ledit polymère : (a) est choisi dans le groupe constitué de : polyéthylénimine (PEI), poly-L-lysine, chitosane, poly-L-arginine, polyamidoamine dendrimérique (PAMAM), polyacrylate, polyphosphate, polyéthylèneglycol (PEG), et toute combinaison de ceux-ci ; ou (b) le poids moléculaire est dans la plage de 500 Da à 60 KDa.

5. Composition selon l'une quelconque des revendications 1 à 4, dans laquelle un diamètre hydrodynamique de ladite particule est dans la plage de 60 nm à 2 µm.

6. Produit pharmaceutique comprenant la composition selon l'une quelconque des revendications 1 à 5, et un support, un excipient ou un adjuvant pharmaceutiquement acceptable.

7. Produit pharmaceutique selon la revendication 6, pour utilisation dans le traitement d'une maladie ou d'un trouble infectieux.

8. Produit pharmaceutique pour utilisation selon la revendication 7, dans lequel la maladie ou le trouble est associé à une infection parasitaire.

9. Produit pharmaceutique pour utilisation selon l'une quelconque des revendications 6 à 8, se présentant sous la forme choisie dans le groupe constitué de : pâte, crème, lotion, mousse, gel, émulsion, pommade et savon.

10. Procédé d'obtention d'une composition comprenant au moins une particule, ayant un noyau comprenant un oxyde métallique, et une enveloppe comprenant un ion d'un élément de lanthanide, dans lequel ledit ion dudit élément de lanthanide a, lié à celui-ci, un polymère et de la pentamidine, le procédé comprenant les étapes consistant à :
a. mélanger un solvant, ledit élément de lanthanide, et une pluralité de particules dudit oxyde métallique, en obtenant ainsi une suspension, et
b. ajouter ledit polymère et ladite pentamidine à ladite suspension.

11. Procédé selon la revendication 10, dans lequel l'étape de mélange est effectuée sous agitation ultrasonore.

12. Procédé selon la revendication 10, dans lequel lesdits ajouts dudit polymère et de ladite pentamidine sont effectués de manière simultanée.

13. Produit pharmaceutique pour utilisation selon la revendication 8, dans lequel ladite infection parasitaire comprend une infection par Leishmania.
